(19) Europäisches Patentamt
European Patent Office
Office européen des brevets

(11) EP 3 997 064 B1

(12) # EUROPÄISCHE PATENTSCHRIFT

(45) Veröffentlichungstag und Bekanntmachung des Hinweises auf die Patenterteilung:
**09.08.2023 Patentblatt 2023/32**

(21) Anmeldenummer: **20735519.9**

(22) Anmeldetag: **25.06.2020**

(51) Internationale Patentklassifikation (IPC):
***C07C 277/08** (2006.01)* ***C07C 279/14** (2006.01)*
***A23K 20/142** (2016.01)*

(52) Gemeinsame Patentklassifikation (CPC):
(C-Sets verfügbar)
**C07C 277/08; C07C 279/14;** C07B 2200/13

(Forts.)

(86) Internationale Anmeldenummer:
**PCT/EP2020/067839**

(87) Internationale Veröffentlichungsnummer:
**WO 2021/008845 (21.01.2021 Gazette 2021/03)**

(54) **VERFAHREN ZUR HERSTELLUNG EINER METASTABILEN KRISTALLMODIFIKATION VON N-(AMINOIMINOMETHYL)-2-AMINOETHANSÄURE (III)**

METHOD FOR PRODUCING A METASTABLE CRYSTAL MODIFICATION OF N-(AMINOIMINOMETHYL)-2-AMINOETHANOIC ACID (III)

PROCÉDÉ DE PRODUCTION D'UNE MODIFICATION CRISTALLINE MÉTASTABLE DE L'ACIDE N- (AMINOIMINOMÉTHYL)-2-AMINOACÉTIQUE (III)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.07.2019 DE 102019118894**
**12.07.2019 DE 102019118893**

(43) Veröffentlichungstag der Anmeldung:
**18.05.2022 Patentblatt 2022/20**

(73) Patentinhaber: **Alzchem Trostberg GmbH**
**83308 Trostberg (DE)**

(72) Erfinder:
- **GÜTHNER, Thomas**
  **83308 Trostberg (DE)**
- **THALHAMMER, Franz**
  **83308 Trostberg (DE)**
- **SANS, Jürgen**
  **83308 Trostberg (DE)**

(74) Vertreter: **Dey, Michael**
**Weickmann & Weickmann**
**Patent- und Rechtsanwälte PartmbB**
**Postfach 860 820**
**81635 München (DE)**

(56) Entgegenhaltungen:
**CN-A- 101 525 305** **DE-C- 964 590**
**US-A- 2 654 779**

- **G. P. JONES ET AL: "Conformations of GABA analogues. I: Crystal and molecular structure of guanidinoacetic acid", JOURNAL OF CRYSTAL AND MOLECULAR STRUCTURE, Bd. 9, Nr. 5, 1. Oktober 1979 (1979-10-01) , Seiten 273-279, XP055716473, US ISSN: 0308-4086, DOI: 10.1007/BF01320842**
- **S. GUHA: "The crystal and molecular structure of glycocyamine", ACTA CRYSTALLOGRAPHICA. SECTION B, STRUCTURAL CRYSTALLOGRAPHY AND CRYSTAL CHEMISTRY., Bd. 29, Nr. 10, 1. Oktober 1973 (1973-10-01), Seiten 2163-2166, XP055716475, DK ISSN: 0567-7408, DOI: 10.1107/S056774087300628X**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

(52) Gemeinsame Patentklassifikation (CPC): (Forts.)

C-Sets
**C07C 277/08, C07C 279/14**

## Beschreibung

Die vorliegende Erfindung betrifft 2 , Kristallgemische sowie ein Verfahren zur Herstellung einer Kristallmodifikation und dieser Kristallgemische.

N-(Aminoiminomethyl)-2-aminoethansäure (CAS-Nr. 352-97-6, Summenformel $C_3H_7N_3O_2$), auch als Guanidinoessigsäure, Guanidinoacetat, Glycocyamin, N-Amidinoglycin oder N-(Aminoiminomethyl)-glycin bekannt, ist eine Guanidinocarbonsäure mit vielfältigen Anwendungen, u.a. in der Synthese von chemischen Produkten, insbesondere Pharmazeutika (vgl. WO 2000/059528), zur direkten Anwendung als pharmazeutischer Wirkstoff bei Nierenerkrankungen (vgl. JP 60054320) oder neurodegenerativen Erkrankungen (vgl. CN 106361736), in der Herstellung von Polymeren (vgl. Du, Shuo et. al., Journal of Materials Science (2018), 53(1), 215-229), als Komplexbildner für Metalle (vgl. Lopes de Miranda et.al., Polyhedron (2003), 22(2), 225-233 bzw. Singh, Padmakshi et. al, Oriental Journal of Chemistry (2008), 24(1), 283-286) und als Zusatzstoff für die Ernährung von Tieren, insbesondere Säugetieren, Fischen, Vögeln (vgl. WO 2005/120246) und dem Menschen (vgl. WO 2008/092591, DE 10 2007 053 369). N-(Aminoiminomethyl)-2-aminoethansäure lässt sich z.B. nach Strecker, M. (Jahresber. Fortschr. Chem. Verw. (1861), 530) aus Glycin durch Umsetzung mit Cyanamid herstellen. Alternativ kann N-(Aminoiminomethyl)-2-aminoethansäure z.B. durch Umsetzung von Glycin mit S-Methylisothioharnstoff-Jodid unter Verwendung von Kaliumhydroxid als Base hergestellt werden (vgl. US 2,654,779).

Auch die Umsetzung von Chloressigsäure mit Ammoniak zu Glycinhydrochlorid und dessen weitere Umsetzung mit Cyanamid wurden beschrieben (vgl. US 2,620,354). Zudem ist die Synthese aus Chloressigsäure und Guanidinhydrochlorid unter Zusatz von Natronlauge bekannt (vgl. CN 101525305).

G.P. Jones und P. J. Pauling, Journal of Crystal and Molecular Structure, Vol. 9, No. 5, 1979, 273-279 beschreiben eine Kristall-und Molekularstruktur von Guanidinoessigsäure. S. Guha, Acta Cryst. (1973), B29, 2163-2166 beschreibt eine Kristall- und Molekularstruktur von Glycocyamin.

Bei den bekannten Verfahren fällt N-(Aminoiminomethyl)-2-aminoethansäure als feinkristallines Pulver an, welches einen erheblichen Staubanteil, d.h. einen erheblichen Anteil an Partikeln, die eine Korngröße kleiner 63 $\mu$m besitzen, aufweist.

Für die Handhabung von chemischen Produkten in fester Form ist es oftmals wünschenswert, dass diese in kristalliner, körniger, rieselfähiger, staubfreier Form ohne oder mit nur geringem Feinkornanteil vorliegen. Insbesondere für die Anwendung als Futtermittelzusatzstoff ist ein schlecht rieselfähiges, staubendes Pulver völlig ungeeignet.

Um diesem Sachverhalt zu begegnen, wurde beispielsweise vorgeschlagen, N-(Aminoiminomethyl)-2-aminoethansäure unter Zusatz von polymeren Bindemitteln (z.B. Methylcellulose) in Mengen von 0,05 bis 15 Gew.-% und Zusatz von Wasser zu Formlingen, Granulaten oder Extrudaten umzuformen (vgl. WO 2009/012960). Nachteilig an diesem Verfahren ist, dass ein Zusatz eines Fremdstoffs, nämlich eines Bindemittels, zwingend erforderlich ist, und dass in einem zusätzlichen Verfahrensschritt, unter Verwendung eines speziellen, technisch aufwendigen und teuren Apparats, wie beispielsweise einem Extruder, Granulator, Intensivmischer oder Pflugscharmischer, und nachfolgender Trocknung das Granulat bzw. die Formlinge hergestellt werden muss.

Nachteilig am Verfahren gemäß obigem Stand der Technik ist zudem, dass Formlinge oder Granulate entweder einen hohen Bindemittelanteil und damit eine geringe Lösungsgeschwindigkeit aufweisen, oder aber bei einem geringen Bindemittelanteil sich zwar relativ schnell auflösen, zugleich aber eine geringe Festigkeit und hohe Abriebwerte aufweisen, so dass die Staubfreiheit nicht mehr gewährleistet werden kann.

Der Erfindung liegt daher die Aufgabe zugrunde, ein Verfahren zur Herstellung von N-(Aminoiminomethyl)-2-aminoethansäure in Form von rieselfähigen, nicht staubenden Kristallaggregaten zur Verfügung zu stellen, welche die Nachteile des Standes der Technik nicht aufweisen, sondern einfach und mit verbreiteten Standardapparaten der chemischen Industrie herstellbar sind, und die zudem eine hohe Löslichkeit aufweisen.

Gelöst werden diese Aufgaben durch ein Verfahren zur Herstellung von N-(Aminoiminomethyl)-2-aminoethansäurevon enthaltend N-(Aminoiminomethyl)-2-aminoethansäure in einer thermodynamisch metastabilen Kristallmodifikation gemäß Anspruch 1 sowie ein Kristallgemisch enthaltend N-(Aminoiminomethyl)-2-aminoethansäure in einer thermodynamisch metastabilen Kristallmodifikation gemäß Anspruch 9. Bevorzugte Ausführungen der Erfindung sind in den Unteransprüchen angegeben, die wahlweise miteinander kombiniert werden können.

Das Auftreten von chemischen Stoffen in verschiedenen Kristallformen bzw. Kristallmodifikationen (Polymorphie) ist sowohl für die Herstellung und Anwendung der Stoffe als auch für die Entwicklung von Formulierungen von großer Bedeutung. So unterscheiden sich die verschiedenen Kristallmodifikationen einer chemischen Verbindung neben dem Aussehen (Kristallhabitus) auch in zahlreichen weiteren physikalischen oder physiko-chemischen Eigenschaften. Es ist bisher nicht möglich, das Auftreten und die Anzahl von Kristallmodifikationen einschließlich ihrer physikalischen oder physiko-chemischen Eigenschaften vorherzusagen. Vor allem die thermodynamische Stabilität und auch das unterschiedliche Verhalten nach Darreichung in lebenden Organismen lassen sich nicht im Voraus bestimmen.

Unter gegebenen Druck- und Temperaturbedingungen haben verschiedene polymorphe Kristallmodifikationen üblicherweise unterschiedliche Gitterenergien bzw. Standardbildungswärmen. Die Kristallform mit der niedrigsten Energie wird als stabile Form bezeichnet. Formen mit höherer energetischer Lage werden, sofern sie isoliert werden können, als metastabil (unter den gegebenen Druck- und Temperaturbedingungen) bezeichnet. Metastabile Polymorphe haben die Tendenz, sich in das stabile Polymorph umzuwandeln. Dies erfordert aufgrund der Metastabilität den Aufwand einer Aktivierungsenergie, z.B. durch Einwirkung von Wärme, mechanischer Energie oder durch Einfluss eines Lösemittels.

Zudem ist allgemein bekannt, dass verschiedene Modifikationen einer Substanz monotrop oder enantiotrop vorliegen können. Im Fall der monotropen Polymorphie kann eine Kristallform bzw. Kristallmodifikation über den gesamten Temperaturbereich bis zum Schmelzpunkt die thermodynamisch stabile Phase darstellen, wohingegen bei enantiotropen Systemen ein Umwandlungspunkt existiert, bei dem sich das Stabilitätsverhältnis umkehrt.

Im Rahmen der vorliegenden Erfindung wurde gefunden, dass N-(Aminoimino-methyl)-2-aminoethansäure neben einer bereits bekannten, thermodynamisch stabilen Kristallmodifikation (im Folgenden auch Form A oder Kristallform A genannt) auch in einer thermodynamisch metastabilen Kristallmodifikation auftritt. Diese hier beschriebene thermodynamisch metastabile Kristallform wird im Folgenden auch Form B oder Kristallform B genannt.

Diese thermodynamisch metastabile Kristallmodifikation (Form B) kann durch einfache Umkristallisation von N-(Aminoiminomethyl)-2-aminoethansäure aus Wasser-haltigen, Guanidinverbindungen enthaltenden Lösungen hergestellt werden. Es hat sich in den zugrundeliegenden Untersuchungen überraschender Weise auch gezeigt, dass diese bisher nicht bekannte, thermodynamisch metastabile Kristallmodifikation von N-(Aminoiminomethyl)-2-aminoethansäure auch mittels der direkten Synthese von N-(Aminoiminomethyl)-2-aminoethansäure in Guanidinverbindungen enthaltenden Lösungen hergestellt werden kann.

Weiterhin überraschend ist, dass diese metastabile Kristallmodifikation Form B bis zu ihrem Schmelzpunkt stabil vorliegt. Eine Feststoffumwandlung von Form B in Form A oder eine reversible Feststoffumwandlung Form A/Form B kann nicht beobachtet werden. Somit liegt mit Form B ein Beispiel für eine monotrope Polymorphie vor.

Somit ist gemäß einer ersten Ausführung der vorliegenden Erfindung ein Verfahren zur Herstellung von N-(Aminoiminomethyl)-2-aminoethansäure enthaltend N-(Aminoiminomethyl)-2-aminoethansäure in einer thermodynamisch metastabilen Kristallmodifikation Gegenstand der vorliegenden Erfindung, wobei die thermodynamisch metastabile Kristallmodifikation im Röntgen-Pulver-Diffraktogramm der Kristallmodifikation bei Verwendung von Cu-K$\alpha$-Strahlung die stärksten Reflexbanden bei $2\Theta$ = 20,2 ° und 23,3 ° und 23,8 ° und 25,3 ° bei einer Messgenauigkeit von +/- 0,2 ° zeigt, in dem N-(Aminoimino-methyl)-2-aminoethansäure aus einer Wasser-haltigen Lösung in Gegenwart von mindestens einer Guanidinverbindungen gemäß Formel (I) kristallisiert wird, wobei für Formel (I) gilt:

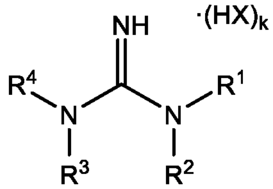

Formel (I)

wobei für die Reste $R^1$, $R^2$, $R^3$, $R^4$ und X, Y sowie den Index k in Formel (I) unabhängig voneinander gilt:

$R^1$, $R^2$ = unabhängig voneinander Wasserstoff, C1- bis C4-Alkyl oder unter Bildung eines Ringes gemeinsam ein Rest der Formel (II), wobei für Formel (II) gilt:

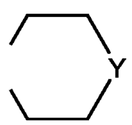

Formel (II)

wobei Y = $CH_2$, O, NH, $NCH_3$ oder eine Bindung bedeutet,
$R^3$, $R^4$ = unabhängig voneinander Wasserstoff oder C1- bis C4-Alkyl,
X = $CH_3COO$, Cl, Br, $NO_3$, $SO_3(NH_2)$ oder ½ $SO_4$,
k = 0 oder 1,

wobei die Guanidinverbindung der Formel (I) in einer Menge von mindestens 8 Gew.-% (bezogen auf das Gesamtgewicht der Lösung) eingesetzt wird.

Die mit diesem Verfahren hergestellte N-(Aminoiminomethyl)-2-aminoethansäure kann als Kristallgemisch, nämlich als Kristallgemisch von Form A und Form B, oder in Reinform, nämlich 100 % Form B vorliegen. Somit ist gemäß

einer weiteren Ausführung der vorliegenden Erfindung auch ein Verfahren zur Herstellung einer thermodynamisch metastabilen Kristallmodifikation von N-(Aminoiminomethyl)-2-aminoethansäure Gegenstand der vorliegenden Erfindung, in dem N-(Aminoiminomethyl)-2-aminoethansäure aus einer Wasser-haltigen Lösung in Gegenwart von mindestens einer Guanidinverbindungen gemäß Formel (I) kristallisiert wird. Mit Hilfe dieses Verfahrens kann N-(Aminoiminomethyl)-2-aminoethansäure in Reinform, nämlich 100 % Form B, bereit gestellt werden.

Des Weiteren ist ein Verfahren zur Herstellung eines Kristallgemisches enthaltend N-(Amino-iminomethyl)-2-aminoethansäure in einer thermodynamisch metastabilen Kristallmodifikation, insbesondere enthaltend N-(Aminoiminomethyl)-2-aminoethansäure in einer thermodynamisch metastabilen Kristallmodifikation und N-(Aminoiminomethyl)-2-amino-ethansäure in einer stabilen Kristallmodifikation, Gegenstand der vorliegenden Erfindung, in dem N-(Aminoiminomethyl)-2-aminoethansäure aus einer Wasser-haltigen Lösung in Gegenwart von mindestens einer Guanidinverbindung gemäß Formel (I) kristallisiert wird.

Gemäß der vorliegenden Erfindung soll dabei unter einem Kristallgemisch ein Gemisch verstanden sein, das N-(Aminoiminomethyl)-2-aminoethansäure in kristalliner Form umfasst, wobei die N-(Aminoiminomethyl)-2-aminoethansäure a) aus Kristallen der Form A und Kristallen der Form B besteht, oder b) aus Kristallen besteht, die mindestens einen ersten Teilbereich aufweisen, der aus Form A besteht und mindestens einen zweiten Teilbereich aufweisen, der aus Form B besteht, oder c) aus Kristallen der Form A und Kristallen der Form B und Kristallen, die mindestens einen ersten Teilbereich aufweisen, der aus Form A besteht und mindestens einen zweiten Teilbereich aufweisen, der aus Form B besteht.

Erfindungsgemäß weist ein Kristallgemisch vorzugsweise mindestens 10 Gew.-%, mehr bevorzugt mindestens 20 Gew.-% und noch mehr bevorzugt mindestens 30 Gew.-% an N-(Aminoiminomethyl)-2-aminoethansäure in Form B auf. Bevorzugt sind Kristallgemische, in denen mindestens 50 Gew.-%, mehr bevorzugt mindestens 75 Gew.-% und noch mehr bevorzugt mindestens 90 Gew.-% der N-(Aminoimino-methyl)-2-aminoethansäure in Form B vorliegt. Form B ist insbesondere charakterisiert durch eine Kristallmodifikation, die im Röntgen-Pulver-Diffraktogramm bei Verwendung von Cu-K$\alpha$-Strahlung diestärksten Reflexbanden bei 2$\Theta$ (2 Theta) = 20,2 ° und 23,3 ° und 23,8 ° und 25,3 ° bei einer Messgenauigkeit von +/- 0,2 ° aufweist.

Der übrige Anteil an N-(Aminoiminomethyl)-2-aminoethansäure liegt in den erfindungsgemäßen Kristallgemischen in einer anderen kristallinen Form vor, vorzugsweise in Form A. Entsprechend umfassen die Kristallgemische vorzugsweise mindestens 10 Gew.-%, vorzugsweise mindestens 20 Gew.-% und mehr bevorzugt mindestens 30 Gew.-% an N-(Aminoiminomethyl)-2-aminoethansäure in der Form A.

Die in diesen Verfahren eingesetzte Wasser-haltige Lösung enthält dabei bevorzugt mindestens 40 Gew.-%, bevorzugt mindestens 45 Gew.-% und besonders bevorzugt mindestens 50 Gew.-% Wasser (bezogen auf das Gesamtgewicht der Lösung).

Ganz besonders bevorzugt kann in dem Verfahren Wasser als Lösungsmittel eingesetzt werden.

Weiterhin bevorzugt kann N-(Aminoiminomethyl)-2-aminoethansäure in einem ersten Verfahrensschritt in Wasser oder einer Wasser-haltigen Lösung gelöst werden, und die N-(Aminoiminomethyl)-2-aminoethansäure enthaltend N-(Aminoiminomethyl)-2-aminoethan-säure in einer thermodynamisch metastabilen Kristallmodifikation in einem zweiten Verfahrensschritt aus der im ersten Verfahrensschritt hergestellten Lösung in Gegenwart der Guanidinverbindung der Formel (I) kristallisiert werden.

Es ist auch möglich, bereits im ersten Verfahrensschritt Wasser oder eine Wasser-haltige Lösung zu verwenden, die eine Guanidinverbindung der Formel (I) enthält.

Alternativ bevorzugt kann das Verfahren jedoch auch derart geführt werden, dass N-(Aminoiminomethyl)-2-aminoethansäure in einem ersten Verfahrensschritt aus Cyanamid und Glycin in Wasser oder in einer Wasser-haltigen Lösung hergestellt wird, und die N-(Aminoiminomethyl)-2-aminoethansäure enthaltend N-(Aminoiminomethyl)-2-aminoethansäure in einer thermodynamisch metastabilen Kristallmodifikation in einem zweiten Verfahrensschritt aus dem im ersten Verfahrensschritt hergestellten Reaktionsgemisch in Gegenwart der Guanidinverbindung der Formel (I) kristallisiert wird.

Somit kann ein Verfahren bereitgestellt werden, indem das gewünschte Produkt direkt ohne eine nachfolgende Umkristallisation gewonnen wird.

Es ist auch möglich, bereits im ersten Verfahrensschritt, also der Umsetzung von Cyanamid und Glycin, Wasser oder eine Wasser-haltige Lösung einzusetzen, die eine Guanidinverbindung der Formel (I) enthält.

Das Produkt der Kristallisation in Gegenwart der Guanidinverbindungen ist eine thermodynamisch metastabile Kristallmodifikation von N-(Aminoiminomethyl)-2-aminoethansäure, die in Reinform im Röntgen-Pulver-Diffraktogramm der Kristallmodifikation bei Verwendung von Cu-K$\alpha$-Strahlung die stärksten Reflexbanden bei 2$\Theta$ (2 Theta) = 20,2 ° und 23,3 ° und 23,8 ° und 25,3 ° bei einer Messgenauigkeit von +/- 0,2 ° zeigt.

Hierbei und im Folgenden bedeutet Cu-K$\alpha$-Strahlung eine Kupfer-K-alpha-Strahlung der Wellenlänge 1,5406 Å, wie sie üblicherweise in kristallographischen Untersuchungen herangezogen wird.

Das Produkt der Kristallisation in Gegenwart der Guanidinverbindungen ist eine thermodynamisch metastabile Kristallmodifikation von N-(Aminoiminomethyl)-2-aminoethansäure, die in Reinform in der orthorhombischen Raumgrup-

pe $P2_12_12_1$ mit Z = 8, d.h. mit zwei kristallographisch unabhängigen Molekülen, kristallisiert und die insbesondere eine pseudo-tetragonale Packung aufweist. Die Elementarzelle weist bei 105 Kelvin die Gitterkonstanten a = 7,7685 Å, b = 7,7683 Å, c = 17,4261 Å bei einer Messgenauigkeit von +/- 0,001 Å auf. Die Einkristall-Messung erfolgte hierbei mit einer Mo-K$\alpha$-Strahlung der Wellenlänge 0,71073 Å bei 105 K (Kelvin).

Gemäß der vorliegenden Erfindung bedeutet eine orthorhombische Raumgruppe eine Raumgruppe, deren Elementarzelle drei rechte Winkel (rechter Winkel = 90 °) aufweist und die drei Kristallachsen a, b und c unterschiedliche Längen aufweisen.

Gemäß einer bevorzugten Ausführung ist damit auch ein Verfahren zur Herstellung einer thermodynamisch metastabilen Kristallmodifikation von N-(Aminoiminomethyl)-2-aminoethan-säure Gegenstand der vorliegenden Erfindung, die in Reinform bevorzugt im Röntgen-Pulver-Diffraktogramm der Kristallmodifikation bei Verwendung von Cu-K$\alpha$-Strahlung die stärksten Reflexbanden bei 2$\Theta$ = 20,2 ° und 23,3 ° und 23,8 ° und 25,3 ° bei einer Messgenauigkeit von +/- 0,2 ° zeigt, und die weiter bevorzugt in der orthorhombischen Raumgruppe $P2_12_12_1$, insbesondere in der orthorhombischen, polaren Raumgruppe $P2_12_12_1$ mit Z = 8 vorliegt und die weiter bevorzugt eine pseudo-tetragonale Packung aufweist. Die Elementarzelle weist bei 105 Kelvin die Gitterkonstanten a = 7,7685 Å, b = 7,7683 Å, c = 17,4261 Å bei einer Messgenauigkeit von +/- 0,001 Å auf. Die Einkristall-Messung erfolgte hierbei mit einer Mo-K$\alpha$-Strahlung der Wellenlänge 0,71073 Å bei 105 K (Kelvin).

Diese Kristallform B bildet bei geeigneten Kristallisationsbedingungen polygonale oder kugelige, radialstrahlige Aggregate aus nadeligen Teilkristalliten, die einen rundlichen Habitus und eine weitgehend einheitliche Aggregatgröße aufweisen. Damit stellen sie eine optimale Handhabung als Feststoff sicher, indem sie ein staubfreies, gut rieselfähiges Produkt ohne Verbackungsneigung ermöglichen. Die Kristallmodifikation B kann als staubarm eingestuft werden, da der Anteil an Kristallen mit einer Korngröße von < 63 $\mu$m (Mesh-Größe) unter 10 %, vorzugsweise unter 5 %, liegt (vgl. Beispiele). Durch ihren Aufbau aus feinen, nadeligen Teilkristalliten stellt dieser Habitus der neuen Kristallform B von N-(Aminoiminomethyl)-2-aminoethansäure zudem eine höhere Lösungsgeschwindigkeit sicher. Zusätzlich und völlig unerwartet bietet N-(Aminoiminomethyl)-2-aminoethansäure der Kristallform B außerdem eine höhere absolute Löslichkeit in Wasser-haltigen Medien.

Wird N-(Aminoiminomethyl)-2-aminoethansäure nach einem der bekannten Verfahren, insbesondere aus Wasser-haltigen Reaktionsmischungen, hergestellt, so fällt die Verbindung in der wohlbekannten Kristallform A an. Ein und dieselbe Kristallstruktur wurde von drei Autorengruppen beschrieben: von Sankarananda Guha, Acta Cryst. B29 (1973), 2163 bzw. von Par J. Berthou et. al., Acta Cryst B32 (1976), 1529 und von Wei Wang et. al, Tetrahedron Letters 56 (2015), 2684. In allen drei Arbeiten wird N-(Aminoiminomethyl)-2-aminoethansäure (hier Form A genannt) beschrieben als monokline Struktur der Raumgruppe $P2_1/n$ mit Z = 4 und den angenäherten Gitterkonstanten a = 4,95 Å, b = 6,00 Å, c = 17,2 Å, $\beta$ = 94,5 °, mit einem Zellvolumen von ca. 510 Å$^3$, wobei bei Berthou et. al. die publizierte Raumgruppe P2,/c über eine Koordinatentransformation zur Raumgruppe $P2_1/n$ überführt wurde. Die experimentelle Kristalldichte von N-(Aminoiminomethyl)-2-aminoethansäure der Form A beträgt ca. 1,50 g/cm$^3$. Das charakteristische Pulverdiffraktogramm von N-(Aminoiminomethyl)-2-aminoethansäure in Form A ist in Figur 1 gezeigt. Unter Verwendung von Cu-K$\alpha$-Strahlung (Kupfer-K-alpha-Strahlung) ist für Form A insbesondere die Bandenlage 2$\Theta$ (2Theta) = 20,7 ° und 26,0 ° charakteristisch. Das Pulverdiffraktogramm stimmt mit dem aus den publizierten Einkristallstrukturdaten berechneten Beugungsmuster überein.

Wird N-(Aminoiminomethyl)-2-aminoethansäure aus üblichen Lösemitteln, wie beispielsweise Wasser, Methanol, Ethanol, Isopropanol oder Mischungen aus Methanol, Ethanol, Ethandiol, oder Acetonitril mit Wasser, kristallisiert, umkristallisiert oder darin hergestellt ohne dass eine Guanidinverbindung gemäß Formel (I) zugegen ist, so fällt N-(Aminoiminomethyl)-2-amino-ethansäure ausschließlich in Kristallform A an, wie durch Versuche gezeigt werden konnte.

Überraschenderweise wurde gefunden, dass N-(Aminoiminomethyl)-2-aminoethansäure bei der Kristallisation aus Wasser oder Wasser-haltigen Lösungen, welches/welche Guanidinverbindungen enthalten, bevorzugt in Kristallform B anfällt. Dies ist umso überraschender, als dass in der bekannten Synthese von N-(Aminoiminomethyl)-2-aminoethansäure aus Chloressigsäure und Guanidinhydrochlorid neben der bekannten Kristallform A keine weiteren Kristallformen gebildet werden (vgl. Beispiele).

N-(Aminoiminomethyl)-2-aminoethansäure der Form B (Reinform) wird charakterisiert durch ihr Pulverdiffraktogramm mit Cu-K$\alpha$-Strahlung (siehe Figur 2), wobei die Banden bei 2$\Theta$ (2Theta) = 20,2 ° und 25,3 ° sowie ein schwächerer Doppelreflex bei 2$\Theta$ (2Theta) = 23,3 °/ 23,8 ° charakteristisch sind. Eine Einkristall-Röntgenstrukturanalyse ergab für N-(Aminoiminomethyl)-2-aminoethansäure der Form B die orthorhombische, polare Raumgruppe $P2_12_12_1$ mit zwei kristallographisch unabhängigen Molekülen, d.h. Z = 8. Die Packung der Moleküle weist eine pseudo-tetragonale Symmetrie auf. Die Elementarzelle weist bei 105 Kelvin die Gitterkonstanten a = 7,7685 Å, b = 7,7683 Å, c = 17,4261 Å bei einer Messgenauigkeit von +/- 0,001 Å auf. Die Einkristall-Messung erfolgte hierbei mit einer Mo-K$\alpha$-Strahlung der Wellenlänge 0,71073 Ä. Das Elementarzellvolumen beträgt 1052 Å$^3$ und die berechnete Röntgen-Kristalldichte 1,479 g/cm$^3$ bei 105 Kelvin.

Die experimentelle Kristalldichte von N-(Aminoiminomethyl)-2-aminoethansäure der Form B beträgt 1,41 g/cm$^3$ +/- 0,03 g/cm$^3$ bei 20 °C. Somit liegt damit die experimentelle Kristalldichte der Form B deutlich unter derjenigen von

Kristallform A, die 1,50 $g/cm^3$ +/- 0,03 $g/cm^3$ bei 20 °C beträgt. Dieser Unterschied in der Kristalldichte deutet auf eine thermodynamische Instabilität von Form B gegenüber Form A hin.

Kristallform B von N-(Aminoiminomethyl)-2-aminoethansäure liegt in Form kugeliger oder polygonaler, radialstrahliger Aggregate mit äußerem rundlichem Habitus vor. Die Einzelkristalle stellen feinste Nadeln dar, aus denen die kugeligen Aggregate aufgebaut sind. Dies hat den überraschenden Vorteil, dass mittels Form B eine physikalische Form von N-(Aminoiminomethyl)-2-aminoethansäure bereitgestellt werden kann, die kugelige oder polygonale, körnige, abriebfeste Aggregate umfasst, mit einer weitgehend einheitlichen Aggregatgröße, einer hervorragenden Rieselfähigkeit und weitgehender Staubfreiheit. Typische Kristallaggregate von N-(Aminoiminomethyl)-2-aminoethansäure der Form B sind in Figur 4 gezeigt. Zum Vergleich wird übliche, dem Stand der Technik entsprechende N-(Aminoimino-methyl)-2-aminoethansäure der Form A, die den Habitus verfilzter, feiner Kristallnadeln aufweist, in Figur 3 gezeigt.

N-(Aminoiminomethyl)-2-aminoethansäure Form A und Form B unterscheiden sich zudem im Infrarotspektrum. Charakteristisch für Form A sind stärkere Banden bei 1005,9, 940,3 und 816,8 $cm^{-1}$, charakteristisch für Form B sind stärkere Banden bei 1148,0, 997,7 und eine nur schwache Bande bei 815 $cm^{-1}$.

Weiterhin zeigen die beiden Kristallformen jeweils in ihrer Reinform unterschiedliche Schmelz- bzw. Zersetzungspunkte:

- N-(Aminoiminomethyl)-2-aminoethansäure Form A:
  DSC onset 280,5 °C, peak 286,3 °C, Schmelzwärme 887 +/- 1 J/g.
- N-(Aminoiminomethyl)-2-aminoethansäure Form B:
  DSC onset 272,5 °C, peak 280,4 °C, Schmelzwärme 860 +/- 1 J/g.

Diese Daten zeigen eindrucksvoll, dass N-(Aminoiminomethyl)-2-aminoethansäure Form B eine thermodynamisch metastabile Kristallmodifikation ist, die im Vergleich zur Form A die thermodynamisch instabilere Form darstellt, wobei der Energieunterschied zwischen beiden Formen ca. 27 J/g beträgt und wobei der Anfangspunkt der Schmelzbereiche (onset) einen Unterschied von 8 K aufweist.

In weitergehenden Untersuchungen hat sich gezeigt, dass N-(Aminoiminomethyl)-2-aminoethansäure Form B eine um ca. 20 % höhere Wasserlöslichkeit als N-(Aminoiminomethyl)-2-aminoethansäure Form A aufweist, und dass dieser Sachverhalt im Temperaturbereich zwischen 5 und 95 °C zutrifft (vgl. Figur 5). Dieser Effekt ist vollkommen unvorhersehbar.

Zusammenfassend ist hier hervorzuheben, dass N-(Aminoiminomethyl)-2-aminoethansäure in der Kristallmodifikation B, insbesondere hergestellt durch Kristallisation von N-(Aminoiminomethyl)-2-aminoethansäure aus einer Guanidinverbindungen enthaltenden Wasser-haltigen Lösung, überraschenderweise vorteilhafte und gewöhnlich entgegengesetzte Eigenschaften, wie z.B. ein grobes, rieselfähiges Korn und zugleich eine hohe Lösungsgeschwindigkeit, die Bildung von Kristallaggregaten ohne Zusatz eines Bindemittels, kombiniert, und eine erhöhte absolute Löslichkeit bei gegebener Temperatur, trotz identischer chemischer Zusammensetzung bereitstellt.

Diese Kristallmodifikation ist aufgrund ihrer hervorragenden Eigenschaften geeignet, als Futtermittelzusatz für Tiere eingesetzt zu werden. Somit ist auch ein Futtermittelzusatz umfassend die hierin beschriebene thermodynamisch metastabile Kristallmodifikation von N-(Aminoiminomethyl)-2-amino-ethansäure Gegenstand der vorliegenden Erfindung. Der Futtermittelzusatz ist insbesondere für Geflügel geeignet.

Derartige Futtermittelzusätze können als Premixe formuliert werden. Somit ist zudem auch die Verwendung der hierin beschriebenen thermodynamisch metastabilen Kristallmodifikation von N-(Aminoiminomethyl)-2-aminoethansäure oder Kristallgemische enthaltend diese Kristallmodifikation zur Herstellung eines Futtermittelzusatzes Gegenstand der Erfindung.

Es hat sich in den zugrundeliegenden Untersuchungen überraschender Weise gezeigt, dass diese bisher nicht bekannte, thermodynamisch metastabile Kristallmodifikation von N-(Aminoiminomethyl)-2-aminoethansäure sowie N-(Aminoiminomethyl)-2-aminoethansäure enthaltend diese thermodynamisch metastabile Kristallmodifikation sowie Kristallgemische enthaltend diese thermodynamisch metastabile Kristallmodifikation, mittels der direkten Synthese von N-(Aminoiminomethyl)-2-aminoethansäure in Guanidinverbindungen enthaltenden Wasser-haltigen Lösungen hergestellt werden können.

Somit ist auch a1) ein Verfahren zur Herstellung einer thermodynamisch metastabilen Kristallmodifikation von N-(Aminoiminomethyl)-2-aminoethansäure sowie a2) ein Verfahren zur Herstellung N-(Aminoiminomethyl)-2-aminoethansäure enthaltend eine thermodynamisch metastabile Kristallmodifikation sowie a3) ein Verfahren zur Herstellung eines Kristallgemisches enthaltend N-(Aminoimino-methyl)-2-aminoethansäure in einer thermodynamisch metastabilen Kristallmodifikation, insbesondere eines Kristallgemisches enthaltend N-(Amino-iminomethyl)-2-aminoethansäure in einer thermodynamisch metastabilen Kristallmodifikation und N-(Aminoiminomethyl)-2-aminoethansäure in einer thermodynamisch stabilen Kristallmodifikation, Gegenstand der vorliegenden Erfindung, in dem N-(Aminoiminomethyl)-2-aminoethansäure in einem ersten Verfahrensschritt aus Cyanamid und Glycin in Wasser oder in einer Wasser-haltigen Lösung hergestellt wird, und a1) die thermodynamisch metastabile Kristallmodifikation, oder a2) die N-(Aminoiminomethyl)-2-aminoethansäure enthaltend N-(Aminoiminomethyl)-2-aminoethansäure in einer thermodynamisch metastabilen Kristallmodifikation, oder a3) das Kristallgemisch, in einem zweiten Verfahrensschritt aus dem im ersten Verfahrensschritt hergestellten Reaktionsgemisch in Gegenwart der Guanidinverbindung der Formel (I) kristallisiert wird.

Die für das erfindungsgemäße Verfahren geeigneten Guanidinverbindungen sind solche der Formel (I)

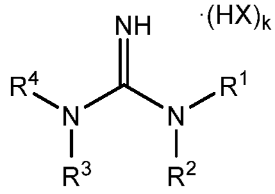

Formel (I)

wobei für die Reste $R^1$, $R^2$, $R^3$, $R^4$ und X, Y sowie den Index k in Formel (I) unabhängig voneinander gilt:

$R^1$, $R^2$ = unabhängig voneinander Wasserstoff, C1- bis C4-Alkyl oder unter Bildung eines Ringes gemeinsam ein Rest der Formel (II), wobei für Formel (II) gilt:

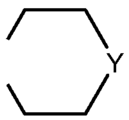

Formel (II)

wobei Y = $CH_2$, O, NH, $NCH_3$ oder eine Bindung bedeutet,
$R^3$, $R^4$ = unabhängig voneinander Wasserstoff oder C1- bis C4-Alkyl,
X = $CH_3COO$, Cl, Br, $NO_3$, $SO_3(NH_2)$ oder ½ $SO_4$,
k = 0 oder 1.

Hierbei bedeutet C1- bis C4-Alkyl insbesondere Methyl, Ethyl, n-Propyl, 2-Methylethyl, n-Butyl, 2-Methyl-propyl oder 1-Methyl-propyl. Besonderes bevorzugt bedeutet C1- bis C4- Alkyl Methyl oder Ethyl.

Weiterhin bevorzugt sind Guanidinverbindungen der Formel (I) wobei mindestens ein Rest $R^1$, $R^2$, $R^3$, $R^4$ ungleich Wasserstoff bedeutet.

Weiterhin bevorzugt können solche Guanidinverbindungen der Formel (I) eingesetzt werden für die gilt:

$R^1$, $R^2$ = unabhängig voneinander Wasserstoff, Methyl oder Ethyl oder unter Bildung eines Ringes gemeinsam ein Rest der Formel (II), wobei für Formel (II) gilt:

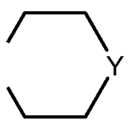

Formel (II)

wobei Y = O, NH oder $NCH_3$ bedeutet,
$R^3$, $R^4$ = Wasserstoff,
X = $CH_3COO$, Cl, Br, $NO_3$, $SO_3(NH_2)$ oder ½ $SO_4$,
k = 0 oder 1.

Hierbei sind solche Verbindungen bevorzugt, für die $(HX)_k$ nicht Hydrochlorid oder Hydrobromid bedeutet. Weiterhin bevorzugt sind Guanidinverbindungen der Formel (I) wobei für den Rest X gilt:
X = $CH_3COO$, $NO_3$, $SO_3(NH_2)$ oder ½ $SO_4$.

Weiterhin bevorzugt können solche Guanidinverbindungen der Formel (I) eingesetzt werden für die gilt:

$R^1$, $R^2$ = unabhängig voneinander Wasserstoff, Methyl oder Ethyl,
$R^3$, $R^4$ = Wasserstoff,
X = $CH_3COO$, Cl, Br, $NO_3$, $SO_3(NH_2)$ oder ½ $SO_4$,
k = 0 oder 1.

Weiterhin bevorzugt können als Guanidinverbindungen Salze eingesetzt werden. Somit können bevorzugt Guanidinverbindungen der Formel (I) eingesetzt werden, für die gilt:
k = 1.

Alternativ bevorzugt können als Guanidinverbindungen ladungsneutrale Verbindungen eingesetzt werden. Somit können bevorzugt Guanidinverbindungen der Formel (I) eingesetzt werden, für die k = 0 gilt. Diese Verbindungen können durch Zugabe von Base in-situ erzeugt werden.

Somit können alternativ bevorzugt auch solche Guanidinverbindungen gemäß Formel (I) eingesetzt werden, für die gilt:

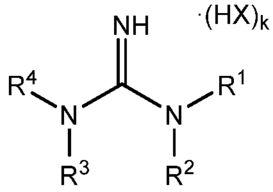

Formel (I)

wobei für die Reste $R^1$, $R^2$, $R^3$, $R^4$ und X, Y sowie den Index k in Formel (I) unabhängig voneinander gilt:

$R^1$, $R^2$ = unabhängig voneinander Wasserstoff, C1- bis C4-Alkyl oder unter Bildung eines Ringes gemeinsam ein Rest der Formel (II), wobei für Formel (II) gilt:

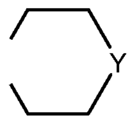

Formel (II)

wobei Y = $CH_2$, O, NH, $NCH_3$ oder eine Bindung bedeutet,
$R^3$, $R^4$ = unabhängig voneinander Wasserstoff oder C1- bis C4-Alkyl,
k = 0.

Die für das Verfahren der Erfindung benötigten Guanidinverbindungen können zum Teil auf bekannten Wegen hergestellt werden. Unsubstituierte Guanidinsalze (d.h. $R^1$=$R^2$=$R^3$=$R^4$=H) können durch Umsetzung von Ammoniumsalzen mit Cyanamid oder Dicyandiamid hergestellt werden. N-substituierte Guanidine bzw. N,N'-disubstituierte Guanidine lassen sich z.B. durch Umsetzung von Aminen oder Aminosäuren mit Cyanamid oder O-Alkylisoharnstoffen herstellen. Höher substituierte Guanidinverbindungen sind durch Umsetzung von Chlor- oder Bromcyan mit Aminen oder Aminosäuren zugänglich. Alternativ können diese Stoffe durch Umsetzung von Thioharnstoffderivaten mit Aminen oder Aminosäuren hergestellt werden.

Die genannten Guanidinverbindungen der Formel (I) können als Einzelstoffe oder in Kombination, insbesondere auch in Kombination von zwei oder mehreren verschiedenen Guanidinverbindungen der Formel (I), eingesetzt werden, um bei der Kristallisation von N-(Aminoiminomethyl)-2-aminoethansäure die Entstehung der thermodynamisch metastabilen Form B zu induzieren. Die Zusatzmengen der genannten Guanidinverbindungen können dabei in weiten Bereichen variieren.

Bevorzugt können die Guanidinverbindungen der Formel (I) in einer Menge eingesetzt werden, die 80 % derjenigen Menge entspricht, die bei 25 °C unter Normaldruck in Wasser maximal gelöst werden kann. Somit kann sichergestellt werden, dass die zur Kristallisation der Form B notwendigen Guanidinverbindungen bei der Kristallisation nicht in das Kristallgefüge einbezogen werden. Weiterhin bevorzugt können die Guanidinverbindungen der Formel (I) in einer Menge von mindestens 8 Gew.-% und höchstens 60 Gew.-% (bezogen auf das Gesamtgewicht der Lösung) eingesetzt werden. Die Guanidinverbindungen der Formel (I) wird in einer Menge von mindestens 8 Gew.-%, und bevorzugt mindestens 10 Gew.-%, weiter bevorzugt mindestens 15 Gew.-%, weiter bevorzugt mindestens 20 Gew.-%, besonders bevorzugt mindestens 25 Gew.-% und ganz besonders bevorzugt mindestens 30 Gew.-%, wobei weiterhin bevorzugt höchstens 60 Gew.-% (jeweils bezogen auf das Gesamtgewicht der Lösung) eingesetzt. Gleichzeitig kann die Guanidinverbindung der Formel (I) in einer Menge bevorzugt von höchstens 50 Gew.-%, weiter bevorzugt von höchstens 40 Gew.-%, bevorzugt höchstens 30 Gew.-%, besonders bevorzugt höchstens 25 Gew.-% und ganz besonders bevorzugt 20 Gew.-% (jeweils bezogen auf das Gesamtgewicht der Lösung) eingesetzt werden.

Bevorzugt ist ein Verfahren zur Herstellung von N-(Aminoiminomethyl)-2-aminoethansäure enthaltend N-(Aminoiminomethyl)-2-aminoethansäure in einer thermodynamisch metastabilen Kristallmodifikation, indem N-(Aminoiminomethyl)-2-aminoethansäure aus einer Wasser-haltigen Lösung in Gegenwart von mindestens einer Guanidinverbindungen der Formel (I) kristallisiert wird, in dem die Guanidinverbindung in einer Menge von mindestens 8 Gew.-% und höchstens 60 Gew.-%, weiter bevorzugt von mindestens 10 Gew.-% und höchstens 60 Gew.-% und ganz besonders bevorzugt von mindestens 15 Gew.-% und höchstens 60 Gew.-%, eingesetzt wird.

Sofern erfindungsgemäß N-(Aminoiminomethyl)-2-aminoethansäure in reiner Form B oder mit einem möglichst hohen Gehalt an Form B erhalten werden soll, werden vorzugsweise die Guanidinverbindungen der Formel (I) in einer Menge von mindestens 30 Gew.%, mehr bevorzugt mindestens 40 Gew.% und noch mehr bevorzugt mindestens 50 Gew.%, jeweils bezogen auf das Gesamtgewicht der Lösung eingesetzt.

Je höher die Konzentration einer definierten erfindungsgemäßen Guanidinverbindung ist, desto höher ist der Gehalt an Form B in dem auskristallisierten Produkt.

Somit ist auch a1) ein Verfahren zur Herstellung einer thermodynamisch metastabilen Kristallmodifikation von N-(Aminoiminomethyl)-2-aminoethansäure sowie a2) ein Verfahren zur Herstellung N-(Aminoiminomethyl)-2-aminoethansäure enthaltend eine thermodynamisch metastabile Kristallmodifikation sowie a3) ein Verfahren zur Herstellung eines Kristallgemisches enthaltend N-(Aminoimino-methyl)-2-aminoethansäure in einer thermodynamisch metastabilen Kristallmodifikation, insbesondere eines Kristallgemisches enthaltend N-(Amino-iminomethyl)-2-aminoethansäure in einer thermodynamisch metastabilen Kristallmodifikation und N-(Aminoiminomethyl)-2-aminoethansäure in einer thermodynamisch stabilen Kristallmodifikation, Gegenstand der vorliegenden Erfindung, in dem eine Wasser-haltige Lösung eingesetzt wird und die Menge an Wasser mindestens 40 Gew.-%, bevorzugt mindestens 45 Gew.-%, weiter bevorzugt mindestens 50 Gew.-% Wasser und besonders bevorzugt höchstens 95 Gew.-% (bezogen auf das Gesamtgewicht der Lösung) beträgt und die Menge an Guanidinverbindung gemäß Formel (I) mindestens 8 Gew.-% und höchstens 60 Gew.-% (bezogen auf das Gesamtgewicht der Lösung) beträgt.

Weiterhin bevorzugt ist ein Verfahren, in dem das Glycin in Wasser oder der Wasser-haltigen Lösung, die mindestens eine Guanidinverbindung der Formel (I) in einer Konzentration von mindestens 8 Gew.-% und höchstens 60 Gew.-% gelöst enthält, vorgelegt wird und das Cyanamid zugegeben wird. Die Zugabe von Cyanamid kann dabei weiter bevorzugt als Feststoff oder in Form einer Lösung, insbesondere einer wässrigen Cyanamid-Lösung, erfolgen.

Als Wasser-haltige Lösungen können solche Lösungen eingesetzt werden, die in Lage sind Glycin und Cyanamid zu lösen.

Die zum Lösen von Glycin vorgelegte wasserhaltige Lösung kann dabei bevorzugt mindestens 40 Gew.-% Wasser sowie ein oder mehrere, insbesondere bis zu 20 verschiedene, Guanidinverbindungen gemäß Formel (I) in Konzentrationen von jeweils einzeln 0,1 bis 60 Gew.-%, so dass die Summe aller Guanidinverbindungen einen Anteil von 8 bis 60 Gew.-% ausmacht. Darin wird Glycin gelöst in einer Konzentration von 3 bis 30 Gew.-% sowie weitere ggf. wasserhaltige Stoffe oder Mischungen (z.B. Natronlauge) zugegeben. Die Mischung vor Dosierung von Cyanamid hat einen Wasseranteil von bevorzugt 35 bis 90 Gew.-%. Cyanamid wird bevorzugt als wässrige Lösung dosiert, insbesondere mit einer Konzentration von 5 bis 60 Gew.-%, bevorzugt 28 bis 52 Gew.-% (bezogen auf die wässrige Cyanamid-Lösung).

Empfehlenswert ist es auch, die Reaktanden der Umsetzung, nämlich Cyanamid und Glycin, bei einer Reaktionstemperatur im Bereich von 20 bis 100 °C, vorzugsweise im Bereich von 60 bis 100 °C, zur Reaktion zu bringen und somit umzusetzen. Dies kann bei Normaldruck, unter Vakuum oder auch unter Druck stattfinden. Bevorzugt kann die Umsetzung bei Normaldruck in einem Temperaturbereich von 20 bis 100 °C erfolgen.

Allerdings empfiehlt es sich auch, die Umsetzung bei einem pH-Wert im Bereich von 7,0 bis 10,0, vorzugsweise bei einem pH-Wert im Bereich von 8,0 bis 10,0 durchzuführen. Der pH-Wert wird mit einer geeigneten Base eingestellt, bei denen es sich sowohl um organische als auch anorganische Basen handeln kann. Bevorzugt können hierbei Natriumhydroxid, Kaliumhydroxid, Calciumhydroxid, Ammoniak oder deren Wasser-haltige Lösungen eingesetzt werden. Besonders bevorzugt können Natriumhydroxid, Calciumhydroxid und deren Wasser-haltige Lösungen eingesetzt werden.

Die Reaktion läuft bei diesen Bedingungen ohne Komplikationen ab, wobei sich das gebildete Produkt nach und nach in dem Reaktionsgemisch bildet und bereits während der Umsetzung auskristallisieren kann. Nach beendeter Zugabe von Cyanamid kann das Reaktionsgemisch noch einige Zeit nachreagieren. Sofern der Sättigungspunkt, d.h. die maximale Konzentration bei der Reaktionstemperatur erreicht ist, beginnt die Kristallisation. Gemäß dem erfindungsgemäßen Verfahren erfolgt die Kristallkeimbildung und Kristallisation vorzugsweise in Form B, wobei die Anwesenheit mindestens einer der genannten Guanidinverbindungen als erfindungswesentlich anzusehen ist. Die Kristallisation des gewünschten Produktes kann bei einer Temperatur im Bereich von -40 bis 100 °C erfolgen.

Überraschenderweise wurde zudem gefunden, dass die Anwesenheit von Guanidinverbindungen der Formel (I) in dem Reaktionsgemisch die Löslichkeit von N-(Aminoiminomethyl)-2-aminoethansäure zum Teil deutlich erhöht, so dass aus der Wasser-haltigen Lösung eine größere Masse N-(Aminoiminomethyl)-2-aminoethansäure, zudem in Kristallform B, erhalten wird. Bevorzugt kann das Verfahren somit durchgeführt werden, indem die N-(Aminoiminomethyl)-2-aminoethansäure in einem Temperaturbereich von -40 bis 100 °C, insbesondere in einem Temperaturbereich von -40 bis 70 °C, weiter bevorzugt in einem Temperaturbereich von -40 bis 50 °C, weiter bevorzugt in einem Temperaturbereich

von -40 bis 40 °C kristallisiert wird.

Besonders bevorzugt ist jedoch ein Verfahren in dem die Kristallisation kontrolliert erfolgt. Hierbei wird das Reaktionsgemisch in konstant gehaltenen Zeitabschnitten definierten Temperaturunterschieden ausgesetzt. Hierdurch kann eine besonders gleichförmige Kristallbildung erreicht werden.

Besonders bevorzugt ist somit ein Verfahren, in dem N-(Aminoiminomethyl)-2-aminoethansäure mit einer Abkühlrate im Bereich von 0,01 bis 5 K/min, weiter bevorzugt im Bereich von 0,1 bis 5 K/min, weiter bevorzugt im Bereich von 0,5 bis 5 K/min, in einem Temperaturbereich von -40 bis 100 °C kristallisiert wird.

In jedem Fall werden vorzugsweise gewöhnliche Rührreaktoren eingesetzt. Der Einsatz aufwendiger verfahrenstechnischer Apparate ist nicht erforderlich.

Nach vollständiger Kristallisation der gewünschten N-(Aminoiminomethyl)-2-aminoethansäure Form B wird das auskristallisierte Produkt vorzugsweise durch Filtration, z.B. mittels Zentrifuge, Druckfilternutsche, Bandfilter oder Filterpresse abfiltriert. Zur Entfernung überschüssiger Reaktanden und Guanidinverbindungen wird vorzugsweise mit dem obengenannten Lösemittel oder Lösemittelgemisch nachgewaschen. Vorzugsweise wird mit Wasser gewaschen, wobei die Temperatur des Waschwassers bevorzugt 0 bis 50 °C beträgt.

Selbstverständlich ist es zur Verbesserung der Wirtschaftlichkeit des Verfahrens möglich, die aus der Abtrennung der N-(Aminoiminomethyl)-2-aminoethansäure der Kristallform B erhaltene Mutterlauge in den Prozess zurückzuführen, ggf. unter Zugabe weiterer Guandinverbindung(en) und/oder durch Konzentrationserhöhung, z.B. durch Eindampfung.

Nach Trocknung, vorzugsweise im Temperaturbereich 40 bis 100 °C, liefert das erfindungsgemäße Verfahren ein trockenes, rieselfähiges, körniges Produkt bestehend aus radialstrahligen, polygonalen oder rundlichen Aggregaten. Die Kristallaggregate haben eine äußere Abmessung, insbesondere eine Mesh-Größe, von 150 bis 3.000 µm, vorzugsweise 300 bis 1.500 µm und einen Staubanteil (d.h. Partikelanteil kleiner 63 µm) von weniger als 5 Gew.-%.

Die so hergestellte N-(Aminoiminomethyl)-2-aminoethansäure Form B hat eine hohe Reinheit, typischerweise > 99,0 %, ist gut handhabbar und zeigt kaum mechanischen Abrieb. Aufgrund dieser Eigenschaften ist die erfindungsgemäße Kristallform B von N-(Aminoiminomethyl)-2-aminoethansäure besonders geeignet für die obengenannten Einsatzzwecke, insbesondere als Zusatzstoff zur Ernährung bzw. als pharmazeutischer Wirkstoff.

Somit ist gemäß einem weiterführenden Gedanken auch die Verwendung a1) der thermodynamisch metastabilen Kristallmodifikation von N-(Aminoiminomethyl)-2-aminoethansäure sowie a2) der N-(Aminoiminomethyl)-2-aminoethansäure enthaltend eine thermodynamisch metastabile Kristallmodifikation sowie a3) des Kristallgemisches enthaltend N-(Aminoiminomethyl)-2-aminoethansäure in einer thermodynamisch metastabilen Kristallmodifikation, insbesondere eines Kristallgemisches enthaltend N-(Aminoiminomethyl)-2-aminoethansäure in einer thermodynamisch metastabilen Kristallmodifikation und N-(Aminoimino-methyl)-2-aminoethansäure in einer thermodynamisch stabilen Kristallmodifikation, jeweils zur Herstellung eines Nahrungsergänzungsmittels oder zur Herstellung eines Futtermittelzusatzes Gegenstand der vorliegenden Erfindung.

Gemäß der vorliegenden Erfindung kann N-(Aminoiminomethyl)-2-aminoethansäure in einer thermodynamisch metastabilen Kristallmodifikation bereitgestellt werden, wobei die N-(Aminoiminomethyl)-2-aminoethansäure vollständig, d.h. zu 100 % in Form B vorliegt.

Somit ist auch ein Verfahren zur Herstellung einer thermodynamisch metastabilen Kristallmodifikation von N-(Aminoiminoethyl)-2-aminoethansäure Gegenstand der vorliegenden Erfindung, wobei die thermodynamisch metastabile Kristallmodifikation die orthorhombische Raumgruppe $P2_12_12_1$ mit Z = 8 mit den Gitterkonstanten a = 7,7685 Å, b = 7,7683 Å und c = 17,4261 Å bei 105 Kelvin und einer Messgenauigkeit von +/- 0,001 Å aufweist.

Alternativ kann N-(Aminoiminomethyl)-2-aminoethansäure gemäß der vorliegenden Erfindung als Kristallgemisch vorliegen, in dem N-(Aminoiminomethyl)-2-aminoethansäure in Form B und in Form A nebeneinander in verschiedenen Kristallen oder innerhalb eines Kristalls nebeneinander kristallisiert ist.

Somit ist auch ein Kristallgemisch Gegenstand der vorliegenden Erfindung, das N-(Aminoiminomethyl)-2-aminoethansäure in einer thermodynamisch metastabilen Kristallmodifikation enthält. Bevorzugt enthält das Kristallgemisch N-(Aminoiminomethyl)-2-aminoethansäure in einer thermodynamisch metastabilen Kristallmodifikation und N-(Aminoiminomethyl)-2-aminoethansäure in einer thermodynamisch stabilen Kristallmodifikation.

Weiter bevorzugt enthält das Kristallgemisch N-(Aminoiminomethyl)-2-aminoethansäure in einer thermodynamisch metastabilen Kristallmodifikation und N-(Amino-iminomethyl)-2-aminoethansäure in einer thermodynamisch stabilen Kristallmodifikation in einem Gewichtsverhältnis im Bereich von 0,1 : 9,9 bis 9,9 : 0,1, weiter bevorzugt im Bereich von 1 : 9 bis 9 : 1, weiter bevorzugt im Bereich von 1 : 1 bis 9 : 1, weiter bevorzugt im Bereich von 2 : 1 bis 9 : 1 und besonders bevorzugt von 4 : 1 bis 9 : 1 enthält.

Diese Kristallgemische sind aufgrund ihrer Kristalleigenschaften hervorragend als Futtermittelzusatz für Zucht- und Masttiere geeignet. Somit ist auch ein Futtermittelzusatz für Zucht- und Masttiere umfassend ein Kristallgemisch enthaltend N-(Aminoiminomethyl)-2-aminoethansäure in einer thermodynamisch metastabilen Kristallmodifikation und N-(Aminoiminomethyl)-2-aminoethansäure in einer thermodynamisch stabilen Kristallmodifikation, Gegenstand der vorliegenden Erfindung.

Besonders bevorzugt ist ein Futtermittelzusatz, in dem das Kristallgemisch die N-(Aminoiminomethyl)-2-aminoethansäure in einer thermodynamisch metastabilen Kristallmodifikation und die N-(Aminoiminomethyl)-2-aminoethansäure in einer thermodynamisch stabilen Kristallmodifikation in einem Gewichtsverhältnis im Bereich von 0,1 : 9,9 bis 9,9 : 0,1, weiter bevorzugt im Bereich von 1 : 9 bis 9 : 1, weiter bevorzugt im Bereich von 1 : 1 bis 9 : 1, weiter bevorzugt im Bereich von 2 : 1 bis 9 : 1 und besonders bevorzugt von 4 : 1 bis 9 : 1 enthält.

Die nachfolgenden Beispiele sollen das Wesen der Erfindung näher erläutern.

Figurenbeschreibung

In den Abbildungen wird gezeigt:

| | |
|---|---|
| Figur 1: | Röntgen-Pulver-Diffraktogramm von N-(Aminoiminomethyl)-2-aminoethansäure der Form A aus Beispiel 1 |
| Figur 2: | Röntgen-Pulver-Diffraktogramm von N-(Aminoiminomethyl)-2-aminoethansäure der Form B aus Beispiel 2.1 |
| Figur 3: | Mikrophotographie von N-(Aminoiminomethyl)-2-aminoethansäure der Form A, hergestellt nach Beispiel 1 (Bildbreite 8 mm) |
| Figur 4: | Mikrophotographie von polygonalen Aggregaten von N-(Aminoiminomethyl)-2-aminoethansäure der Form B, hergestellt durch Umkristallisation aus einer 50 %-igen Wasser-haltigen Guanidinhydrochloridlösung gemäß Beispiel 2.1 (Bildbreite 8 mm) |
| Figur 5: | Löslichkeitskurve von N-(Aminoiminomethyl)-2-aminoethansäure der Form A bzw. Form B in Wasser |
| Figur 6: | Abbildung der beiden kristallographisch unabhängigen Moleküle N-(Aminoiminomethyl)-2-aminoethansäure aus der Einkristall-Röntgenstrukturanalyse |
| Figur 7: | Abbildung der Packung der Moleküle von N-(Aminoiminomethyl)-2-aminoethansäure im Kristallverband. Die Blickrichtung ist entlang der a-Achse. Deutlich sind voneinander unabhängige, senkrecht zueinander angeordnete, über H-Brücken gebundene Molekülketten parallel der a- und der b-Achse zu sehen. Diese Ketten sind entlang der c-Achse gestapelt. |
| Figur 8: | Schüttdichte abhängig von der Konzentration Form B. |
| Figur 9: | Eichkurve Anteil Kristallform B zu Kristallform A. |
| Figur 10: | DSC N-(Aminoiminomethyl)-2-aminoethansäure Form A. |
| Figur 11: | DSC N-(Aminoiminomethyl)-2-aminoethansäure Form B. |

Beispiele

Verwendete Guandinverbindungen

Technisch verfügbare Guanidinverbindungen wurden im Handel bezogen:

Guanidinhydrochlorid: Sigma-Aldrich Best.-Nr. G4505
Guanidinsulfamat: TCI Chemicals Best.-Nr. G0022
Guanidinnitrat: Sigma-Aldrich Best.-Nr. 234249
Guanidinacetat: Sigma-Aldrich Best.-Nr. 50920
N,N'-Dimethylguanidinhydrochlorid: AlzChem AG
N,N'-Dimethylguanidinsulfat: Sigma-Aldrich Best.-Nr. 276669
N,N'-Diethylguanidinsulfat: TCI Chemicals Best.-Nr. D2035,
1-(Aminoiminomethyl)-4-methyl-piperazin-Sulfat: ABCR GmbH, Best.-Nr. AB288540
4-(Aminoiminomethyl)-morpholin-Acetat: ABCR GmbH, Best.-Nr. AB301888
N-Methylguanidinsulfat: TCI Chemicals, Best.-Nr. M1691
N-Ethylguanidinsulfat: Sigma-Aldrich, Best.Nr. 275557

Röntgen-Pulver-diffraktometrische Messung

Im Umfang der vorliegenden Beispiele wurden Röntgen-Pulver-diffraktometrische Messungen unter Verwendung eines Pulver-Diffraktometers Bruker D2 Phaser mit Theta/2Theta-Geometrie, einem LYNXEYE-Detektor, Cu-K$\alpha$-Strahlung der Wellenlänge 1,5406 Å mit einer Beschleunigungsspannung von 30 kV und einem Anodenstrom von 10 mA, einem Nickelfilter und einer Schrittweite von 0,02 ° durchgeführt. Die zur Untersuchung stehenden Proben wurden im Achatmörser vermahlen und gemäß Herstellerangaben auf den Probenteller gedrückt und die Oberfläche geglättet.

Eichgerade für die röntgenographische Bestimmung des Anteils Form A / B

Mit mechanischen Mischungen von reinen Proben GAA Form A und Form B wurden XRD-Daten ermittelt. Die Peakhöhen bei 20,7 ° bzw. 20,2 ° wurden zur quantitativen Auswertung über die Peakhöhe herangezogen. Die daraus ermittelte Eichkurve (Kalibriergerade) mit sehr gutem Korrelationskoeffizient wurde für unbekannte Proben zur Bestimmung des Anteils Form A/B herangezogen (vgl. Tabelle 1 und Figur 9).

Tabelle 1: Eichkurve Anteil Kristallform A zu Kristallform B

| Gewichtsanteil Form B (%) | Gewichtsanteil Form A (%) | Röntgen Zählrate Form A (20,7 °) | Röntgen Zählrate Form B (20,2 °) | Verhältnis Zähl rate B zu A+B |
|---|---|---|---|---|
| 0 | 100 | 4000 | 1 | 0,00025 |
| 5 | 95 | 3150 | 150 | 0,04545 |
| 10 | 90 | 3500 | 500 | 0,12500 |
| 20 | 80 | 3500 | 900 | 0,20455 |
| 33 | 67 | 3050 | 1600 | 0,34409 |
| 50 | 50 | 2400 | 2400 | 0,50000 |
| 67 | 33 | 1750 | 3100 | 0,63918 |
| 80 | 20 | 950 | 3250 | 0,77381 |
| 90 | 10 | 400 | 3700 | 0,90244 |
| 95 | 5 | 250 | 4000 | 0,94118 |
| 100 | 0 | 1 | 4100 | 0,99976 |

Zur quantitativen Bestimmung des Verhältnisses von N-(Aminoiminomethyl)-2-amino-ethansäure Kristallform B zu Kristallform A wurden mechanische Mischungen von pulverförmigen Proben der jeweils reinen Kristallformen hergestellt und am Röntgen-Pulverdiffraktometer vermessen. Die Mischungsverhältnisse waren 100 : 0, 95 : 5, 90 : 10, 80 : 20, 67 : 33, 50 : 50, 33 : 67, 20 : 80, 10 : 90, 5 : 95 und 0 : 100. Die Signalhöhen (Zählraten) bei 2Theta 20,2 ° (Form B) wurden zur Summe der Signalhöhen bei 2Theta 20,7 ° (Form A) und 2Theta 20,2 ° (Form B) ins Verhältnis gesetzt und daraus eine Kalibriergerade ermittelt. Mit einem Korrelationskoeffizienten $R^2$=0,998 wurde folgender linearer Zusammenhang gefunden:

$$\text{Gewichtsprozent Form B} = \left(\left(\frac{\text{Zählrate Form B}}{\text{Zählrate Form A} + \text{Zählrate Form B}}\right) - 0{,}0074\right) * 102{,}04$$

Diese Formel wurde in den nachfolgenden Beispielen zur Bestimmung des jeweiligen Anteils Kristallform A und Kristallform B herangezogen.

Einkristall-Röntgenstrukturanalyse

Ein geeigneter Kristall wurde durch Verdunsten einer Wasser-haltigen Lösung von N-(Aminoiminomethyl)-2-aminoethansäure in Gegenwart von N,N'-Guanidinodiessigsäure hergestellt. Die Einkristallmessung erfolgte bei 105 Kelvin an einem Kristall der Dimension 0,02 * 0,02 * 0,09 mm unter Verwendung monochromatischer Mo-K$\alpha$ (Molybdän-K-alpha)-Strahlung der Wellenlänge 0,71073 Å unter Einsatz eines Zweikreis-Diffraktometers Bruker D8 Venture TXS. Die Verfeinerung der Röntgenkristalldaten unter Verwendung von 2072 unabhängigen Reflexen erfolgte nach der Methode der kleinsten Fehlerquadrate bis zu einem R-Wert ($F_{obs}$) von 0,0381. Die Position der NH- und OH-Wasserstoffatome wurde verfeinert, die der CH-Wasserstoffatome an der berechneten Position fixiert. Das Ergebnis der Röntgen-Einkristallstrukturanalyse ist in Figur 6 und 7 veranschaulicht. Ein aus der Einkristallstrukturanalyse rückgerechnetes Pulverdiffraktogramm stimmte exakt mit dem gemessenen Pulverdiffraktogramm gemäß Figur 2 überein.

Beispiel 1 (Vergleich) - Umkristallisation von N-(Aminoiminomethyl)-2-aminoethansäure aus Wasser

400 g Wasser wurden bei 80 °C vorgelegt und löffelweise insgesamt 11,66 g N-(Aminoiminomethyl)-2-amino-

ethansäure mit einem Gehalt von 99,0 %, vorliegend in Kristallform A, darin gelöst, wobei mit der letzten Portion die Löslichkeitsgrenze überschritten wurde. Dann wurde bei 80 °C abfiltriert, das Filtrat mit weiteren 100 g Wasser versetzt und auf 80 °C erhitzt. Es bildete sich eine knapp gesättigte, klare Lösung. Durch langsame Abkühlung auf 20 °C innerhalb von 4 Stunden wurde N-(Aminoiminomethyl)-2-aminoethansäure kristallisiert. Die ausgefallenen Kristalle wurden abfiltriert und bei 60 °C im Vakuum getrocknet. Es wurden 6,51 g N-(Aminoiminomethyl)-2-aminoethansäure mit einem Gehalt von 99,1 % erhalten.

Das erhaltene Produkt liegt in Form fein-nadeliger Kristalle vor. Die fein-nadeligen Kristalle wurden mikroskopisch untersucht (siehe Figur 3). Eine Röntgen-Pulver-diffraktometrische Messung ergab das mit Figur 1 gezeigte Pulverdiffraktogramm, welches die altbekannte Kristallform A anzeigt.

Beispiel 2 - Kristallisation von N-(Aminoiminomethyl)-2-aminoethansäure aus Guanidinhydrochlorid-Lösungen

Aus 250 g Guanidinhydrochlorid und 250 g Wasser wurde eine 50 %-ige Lösung hergestellt. In 400 g dieser Lösung wurde bei 80 °C löffelweise N-(Aminoiminomethyl)-2-aminoethansäure derselben Zusammensetzung wie in Beispiel 1 zugesetzt. Erst bei einer zugegebenen Menge von 26,37 g war die Löslichkeitsgrenze überschritten. Der geringe Feststoffanteil wurde bei 80 °C abfiltriert, das Filtrat mit den restlichen 100 g der 50 %-igen Lösung von Guanidinhydrochlorid versetzt und bei 80 °C für 1 Stunde gerührt. Es wurde eine klare, farblose Lösung erhalten. Durch langsame Abkühlung auf 20 °C innerhalb von 4 Stunden wurde N-(Aminoiminomethyl)-2-aminoethansäure kristallisiert. Die ausgefallenen Kristallaggregate wurden abfiltriert, 3 mal mit Wasser von 20 °C gewaschen und bei 60 °C getrocknet. Es wurden 15,56 g N-(Aminoiminomethyl)-2-aminoethansäure mit einem Gehalt von 99,2 % erhalten. Die erhaltene Menge ist somit um den Faktor 2,4 größer als in Beispiel 1, was auf die durch die Guanidinhydrochloridlösung deutlich erhöhte Löslichkeit von N-(Aminoiminomethyl)-2-aminoethansäure zurückzuführen ist.

Ein analog aufgenommenes Pulverdiffraktogramm (siehe Figur 2) zeigte die bislang unbekannte Kristallform B an. Die polygonalen, rundlichen Kristallaggregate wurden mikroskopisch untersucht (siehe Figur 4).

Der Versuch wurde in analoger Weise mit unterschiedlichen Konzentrationen von Guanidinhydrochlorid in Wasser wiederholt. Dabei zeigte sich, dass mit fallender Konzentration zunehmend N-(Aminoiminomethyl)-2-aminoethansäure der Form A entstand (vgl. Tabelle 2). Der Konzentration der eingesetzten Guanidinverbindung im zur Umkristallisation von N-(Aminoiminomethyl)-2-aminoethansäure eingesetzten Lösemittel kommt also entscheidende Bedeutung zu.

Tabelle 2: Kristallisation aus Guanidinhydrochlorid-Lösungen verschiedener Konzentration

| Nr. | Konzentration Guanidinhydrochlorid | Auswaage | Kristallform |
|---|---|---|---|
| 2.1 | 50 Gew-% | 15,56 g | 100 % Form B |
| 2.2 | 40 Gew-% | 12,43 g | 61 % Form B<br>39 % Form A |
| 2.3 | 30 Gew-% | 10,64 g | 16 % Form B<br>84 % Form A |
| 2.4 | 20 Gew-% | 9,63 g | 100 % Form A |
| 2.5 | 10 Gew-% | 9,43 g | 100 % Form A |

Beispiel 3 - Umkristallisation von N-(Aminoiminomethyl)-2-aminoethansäure aus Wasser-haltigen Lösungen weiterer Guanidinverbindungen

Analog zu Beispiel 2 wurden wässrige Lösungen von verschiedenen Guandinverbindungen in den jeweils angegebenen Konzentrationen (C) hergestellt. In 400 g der jeweiligen Lösung wurde bei 80 °C die jeweils angegebene Menge (M) von N-(Aminoiminomethyl)-2-aminoethansäure gelöst. Nach Filtration bei 80 °C wurden weitere 100 g der angegebenen wässrigen Lösung der jeweiligen Guanidinverbindung zugesetzt und die klare Lösung 1 Stunde bei 80 °C gerührt. Durch langsame Abkühlung auf 20 °C innerhalb von 4 Stunden wurde N-(Aminoiminomethyl)-2-aminoethansäure kristallisiert. Die ausgefallenen Kristallaggregate wurden abfiltriert, 3 mal mit Wasser von 20 °C gewaschen und bei 60 °C getrocknet. Es wurde die jeweils angegebene Menge (A) N-(Aminoiminomethyl)-2-aminoethansäure mit einem Gehalt (G) erhalten (vgl. Tabelle 3a/b)

Von den jeweiligen Produkten wurden Pulverdiffraktogramme aufgenommen und auf das Vorliegen der jeweiligen Kristallformen untersucht, wobei die oben angegebene Formel zur Bestimmung der Mengenanteile von Form A und Form B angewendet wurde.

Tabelle 3a: Umkristallisation aus Wasser-haltigen Lösungen von Guanidinverbindungen (nicht erfindungsgemäß)

| Nr. | Guandinverbindung | C Gew.-% | M g | A g | G % | Kristallform |
|---|---|---|---|---|---|---|
| 3.1 | Melamin | 0,3 | 16,98 | 11,13 | 99,3 | 100 % Form A |
| 3.2 | N-Cyanoguanidin | 3,0 | 19,80 | 10,09 | 99,1 | 100 % Form A |
| 3.3 | L-Arginin | 10,0 | 18,37 | 11,98 | 99,2 | 100 % Form A |
| 3.4 | N-(Aminoimino-methyl)-N-methyl-2-aminoethansäure | 1,0 | 17,47 | 8,14 | 99,2 | 100 % Form A |
| 3.5 | Biguanid-Hydrochlorid | 50,0 | 13,72 | 7,13 | 99,3 | 100 % Form A |
| 3.6 | N,N-Dimethylbiguanid | 30,0 | 10,06 | 5,81 | 99,1 | 100 % Form A |
| 3.7 | N,N-Dimethylbiguanid-Hydrochlorid | 2,0 | 22,30 | 11,89 | 99,3 | 100 % Form A |

Tabelle 3b: Umkristallisation aus Wasser-haltigen Lösungen von Guanidinverbindungen gemäß Formel (I) - erfindungsgemäß

| Nr. | Guandinverbindung | C Gew.-% | M g | A g | G % | Kristallform |
|---|---|---|---|---|---|---|
| 3.10 | Guanidin-Nitrat | 15 | 18,63 | 15,29 | 98,8 | 52 % Form B<br>48 % Form A |
| 3.11 | Guanidin-Amidosulfonat | 40 | 20,71 | 14,77 | 99,0 | 78 % Form B<br>22 % Form A |
| 3.12 | Guanidin-Acetat | 50 | 17,39 | 7,89 | 99,2 | 94 % Form B<br>6 % Form A |
| 3.13 | N,N-Dimethylguanidin-Hydrochlorid | 50 | 16,18 | 8,57 | 99,0 | 36 % Form B<br>64 % Form A |
| 3.14 | N,N-Dimethylguanidin-Sulfat | 30 | 30,13 | 15,61 | 99,1 | 51 % Form B<br>49 % Form A |
| 3.15 | N,N-Diethylguanidin-Hydrochlorid | 50 | 27,11 | 13,56 | 99,2 | 100 % Form B |
| 3.16 | N-Methylguanidin-Sulfat | 50 | 34,65 | 18,14 | 98,9 | 100 % Form B |
| 3.17 | N-Ethylguanidin-Sulfat | 50 | 40,22 | 20,47 | 99,0 | 69 % Form B<br>31 % Form A |
| 3.18 | 1-(Aminoiminomethyl)-4-methyl-piperazin-Sulfat | 30 | 21,84 | 14,70 | 99,1 | 100 % Form B |
| 3.19 | 4-(Aminoiminomethyl)-morpholin-Acetat | 30 | 18,11 | 10,02 | 99,3 | 100 % Form B |

Die erfindungsgemäßen Guanidinverbindungen gemäß Formel (I) induzieren bei der Kristallisation von N-(Aminoiminomethyl)-2-aminoethansäure somit bevorzugt Kristallform B, wobei in manchen Fällen auch Gemische mit Form A auftreten (vgl. Tabelle 3b). Andere, nicht erfindungsgemäße Guanidine sind hierzu nicht in der Lage (vgl. Tabelle 3a).

Beispiel 4 (Vergleich) - Synthese von N-(Aminoiminomethyl)-2-aminoethansäure aus Glycin und Cyanamid in wässriger Lösung

112,6 g (1,5 mol) Glycin wurden in 300 g Wasser gelöst. Die Lösung wurde mit 21,6 g (0,27 mol) einer 50 %-igen Natronlauge versetzt wobei sich ein pH-Wert von 8,4 ergab. Bei 80 °C wurde im Verlauf von 4 Stunden eine Lösung von 42,04 g (1,0 mol) Cyanamid gelöst in 42 g Wasser zudosiert. Die Nachreaktion erfolgte für eine weitere Stunde bei 80 °C. Die erhaltene Suspension wurde auf 20 °C abgekühlt, abfiltriert, mit Wasser gewaschen und bei 60 °C getrocknet. Es wurden 100,6 g N-(Aminoiminomethyl)-2-aminoethansäure mit einem Gehalt von 99,1 % erhalten. Die Ausbeute

betrug 85,9 %.

Ein Pulverdiffraktogramm der erhaltenen fein-nadeligen Kristalle zeigte die alleinige Anwesenheit von Form A (100 % Form A) an.

Beispiel 5 (Vergleich) - Synthese von N-(Aminoiminomethyl)-2-aminoethansäure aus Chloressigsäure und Guanidinhydrochlorid

Wie in CN 101525305 beschrieben wurden 200 g NaOH (fest, 5,0 mol) in 600 g Wasser gelöst. Bei maximal 15 °C wurden 480 g (5,03 mol) festes Guanidinhydrochlorid eingetragen. Es wurde eine Wasser-haltige Lösung der Guanidinbase erhalten. 460 g (4,87 mol) Chloressigsäure wurden in 460 g Wasser gelöst. Dazu wurden 760 g einer 25 %igen Natronlauge (entsprechend 4,75 mol NaOH) über 1 Stunde bei 20 °C dosiert. Es wurde eine Wasser-haltige Lösung von Natrium-Chloracetat erhalten. Die Guanidinlösung wurde über 2 Stunden zu der auf 40 °C erwärmten Lösung des Natrium-chloracetats dosiert. Die Nachreaktion erfolgte bei 40 °C über einen Zeitraum von 24 Stunden. Die erhaltene weiße Suspension wurde filtriert, mit Wasser gewaschen und bei 60 °C getrocknet. Es wurden 404 g (71 %) GAA mit einem Gehalt von 98 % erhalten. Eine röntgenpulverdiffraktometrische Analyse ergab, dass die erhaltene GAA zu 100 % in Kristallform A vorlag. Trotz der Anwesenheit von Guanidin(-base) als Reaktionspartner reicht deren Konzentration nicht aus, die Kristallform von GAA zu beeinflussen. Dies steht in Einklang mit Beispiel 2, da ja erst ab einer Konzentration von 30 Gew-% Guanidinhydrochlorid ein meßbarer Effekt gesehen wurde. Derartige Konzentrationen werden bei der Herstellweise gemäß CN 101525305 nicht erreicht, so dass für derartig hergestellte Produkte ausschließlich Kristallform A anzunehmen ist.

Beispiel 6 (erfindungsgemäß) - Synthese von N-(Aminoiminomethyl)-2-aminoethansäure aus Glycin und Cyanamid in einer 50 %-igen Lösung von Guanidinhydrochlorid

Aus 150 g Guanidinhydrochlorid und 150 g Wasser wurde eine Lösung hergestellt. Darin wurden 112,6 g (1,5 mol) Glycin gelöst und mit 22,4 g (0,28 mol) einer 50 %-igen Natronlauge ein pH-Wert von 8,4 eingestellt. Bei 80 °C wurde im Verlauf von 4 Stunden eine Lösung von 42,04 g (1,0 mol) Cyanamid gelöst in 42 g Wasser zudosiert. Die Nachreaktion erfolgte für eine weitere Stunde bei 80 °C. Die erhaltene Suspension wurde auf 20 °C abgekühlt, abfiltriert, mit Wasser gewaschen und bei 60 °C getrocknet. Es wurden 100,8 g N-(Aminoiminomethyl)-2-aminoethansäure mit einem Gehalt von 99,1 % erhalten. Die Ausbeute betrug 86,0 %.

Ein Pulverdiffraktogramm der erhaltenen rundlichen Kristallaggregate aus radialstrahligen Einzelkristallen zeigte die alleinige Anwesenheit von Form B (100 % Form B) an.

Beispiel 7 - Physikalisch-chemische Charakterisierung von N-(Aminoiminomethyl)-2-aminoethansäure der Form A und der Form B

7.1 Schmelz- bzw. Zersetzungspunkt

Zur Dynamischen Differential Scanning Calorimetry (DSC) wurde ein Gerät Mettler DSC 3+ mit 40 μl Aluminiumtiegel eingesetzt. Die Heizrate betrug 10 Kelvin pro Minute bei einem Temperaturbereich von 30 bis 350 °C. Jeweils ca. 1,4 mg der Produkte aus Beispiel 1 und 2.1 wurden in Aluminium-Tiegel eingewogen und bei Atmosphärendruck (960 mbar bei einer Höhenlage von 500 m über NN) vermessen.

Die Probe aus Beispiel 1 (= N-(Aminoiminomethyl)-2-aminoethansäure der Form A) zeigte einen onset (Wendepunkt der Schmelzkurve projiziert auf die Basislinie) von 280,5 °C und eine Peaktemperatur der Schmelzkurve von 286,3 °C. Die gesamte endotherme Schmelzwärme betrug 887 J/g (vgl. Figur 10). Das Produkt verfärbte sich beim Schmelzen von weiß nach braun.

Die Probe aus Beispiel 2.1 (= N-(Aminoiminomethyl)-2-aminoethansäure Form B) wurde anlog vermessen. Sie zeigte einen onset von 272,5 °C und einen Peak bei 280,4 °C, die Schmelzwärme betrug 860 J/g, die Verfärbung war identisch (vgl. Figur 11).

Form B schmilzt demnach ca. 6 bis 8 Kelvin tiefer als Form A und hat eine um 27 J/g niedrigere Schmelzwärme bzw. eine um 27 J/g höhere Gitterenergie. Anders ausgedrückt werden für Form B 27 J/g weniger Energie benötigt als für Form A, um den energiegleichen Schmelzzustand zu erreichen. Form B stellt damit eine metastabile Kristallform bzw. ein unter normalen Druck- und Temperaturbedingungen energetisch höherliegendes Polymorph von N-(Aminoiminomethyl)-2-aminoethansäure dar.

Diese neue metastabile Kristallmodifikation Form B liegt bis zu ihrem Schmelzpunkt stabil vor. Eine Feststoffumwandlung von Form B in Form A oder eine reversible Feststoffumwandlung Form A/Form B kann nicht beobachtet werden. Somit liegt mit Form B ein Beispiel für eine monotrope Polymorphie vor.

7.2 Bestimmung der Wasserlöslichkeit

100 g Wasser von 5 °C wurden vorgelegt. Darin wurde das Produkt aus Beispiel 1 (= N-(Aminoiminomethyl)-2-aminoethansäure Form A) bis zur Sättigung gelöst und die gelöste Menge durch Rückwägung bestimmt. Dann wurde die Temperatur auf 20 °C erhöht und so viel der Probe zugegeben, bis wieder der Sättigungspunkt erreicht war. Dasselbe wurde bei weiteren Temperaturen, maximal bei 95 °C, wiederholt. Eine analoge Messung wurde mit dem Produkt aus Beispiel 2.1 (= N-(Aminoiminomethyl)-2-aminoethansäure Form B) durchgeführt. Die erhaltenen Löslichkeitsdaten für beide Produkte wurden graphisch in Figur 5 zusammengefasst.

Beide Kristallformen von N-(Aminoiminomethyl)-2-aminoethansäure lösen sich mit steigenden Temperatur besser in Wasser. Die erfindungsgemäße N-(Aminoiminomethyl)-2-aminoethan-säure Form B löst sich bei jeder Temperatur um ca. 20 % besser als die bekannte Form A.

7.3 Bestimmung der Dichte

Kristalle von N-(Aminoiminomethyl)-2-aminoethansäure Form A aus Beispiel 1 wurden bei 20 °C in Tetrachlormethan eingebracht, wo sie an er Oberfläche schwammen. Durch tropfenweiser Zugabe von Dichlormethan wurde die Dichte des flüssigen Mediums so lange erniedrigt, bis die Kristalle gerade eben in der Flüssigkeit zu schweben kamen, ohne aufzusteigen und ohne auf den Boden abzusinken. Die Dichte der flüssigen Phase wurde im Pyknometer bestimmt. Es wurden 1,50 +/- 0,03 g/cm$^3$ gemessen.

Analog wurde mit Kristallen der Form B aus Beispiel 2.1 verfahren. Die Dichte bei 20 °C wurde zu 1,41 +/- 0,03 g/cm$^3$ bestimmt.

Form B hat demnach eine um 6 % geringere Dichte als Form A. Dies korreliert mit der oben bestimmten niedrigeren Gitterenergie von Form B. Die gemessenen Kristalldichten stimmen zudem mit den aus den jeweiligen Gitterkonstanten berechneten Röntgen-Kristalldichten überein.

7.4 Bestimmung des Staubanteils

Das Produkt aus Beispiel 1 wurde über ein Sieb mit Maschenweite 63 μm (entspricht 230 Mesh - Mesh-Größe) abgesiebt. Es wurden 46 Gew.-% Feinanteil erhalten. Analog wurde mit der aus polygonalen, rundlichen Kristallaggregaten bestehenden Probe aus Beispiel 2.1 verfahren. Hier wurde ein Feinanteil von unter 3 Gew.-% bestimmt. Staubarme, damit sicher handhabbare Materialien sollten einen Staubanteil (d.h. Kornanteil < 63 μm) von unter 10 % aufweisen. Das Produkt aus Beispiel 2.1 (N-(Aminoiminomethyl)-2-aminoethansäure der Kristallform B) erfüllt dies, während das Vergleichsbeispiel 1 (N-(Aminoiminomethyl)-2-aminoethansäure der Kristallform A) dies nicht erfüllt.

7.5 Bestimmung des Schüttwinkels

Das Produkt aus Beispiel 1, bestehend aus ineinander verfilzten nadeligen Kristallen, wurde mit einer Vorrichtung nach DIN ISO 4324 durch einen Trichter auf eine ebene Fläche geschüttet. Nach Entfernen des Trichters wurde der Böschungswinkel des erhaltenen Kegels mit einer Winkelmesseinrichtung bestimmt. Dieser betrug ca. 45 °. N-(Aminoiminomethyl)-2-aminoethansäure Form A zeigt demnach ein schlechtes Fließverhalten. Das körnige Produkt aus Beispiel 2.1 wurde anlog vermessen. Hier wurde ein Böschungswinkel von ca. 25 ° erhalten. N-(Aminoiminomethyl)-2-aminoethansäure Form B zeigt demnach ein hervorragendes Fließverhalten.

7.6 Bestimmung der Schüttdichte

Eine eingewogene Menge des Produkts aus Beispiel 1 wurde in einen Messzylinder gegeben und durch zweimaliges festes Aufklopfen auf den Labortisch partiell verdichtet. Aus der Füllhöhe des Messzylinders wurde die Schüttdichte zu 0,37 g/cm$^3$ bestimmt. Analog wurde mit dem Produkt aus Beispiel 2.1 verfahren. Hier wurde eine Schüttdichte von 0,62 g/cm$^3$ bestimmt. N-(Aminoiminomethyl)-2-aminoethansäure der Form B hat somit eine deutlich erhöhte Schüttdichte, was für Verpackung, Transport und Handhabung des Produkts vorteilhaft ist.

7.7 Thermische Stabilität von N-(Aminoiminomethyl)-2-aminoethansäure Form B

a) N-(Aminoiminomethyl)-2-aminoethansäure Form B aus Beispiel 2.1 wurde für 6 Stunden bei 120 °C in den Trockenschrank gestellt. Anschließend wurde mittels Röntgen-Pulver-Diffraktometrie die Kristallform bestimmt. Diese blieb unverändert reine Kristallform B.

b) N-(Aminoiminomethyl)-2-aminoethansäure Form B aus Beispiel 2.1 wurde mit 20 % Wasser angefeuchtet, für 6 Stunden bei 65 °C in einem geschlossenen Gefäß inkubiert, dann getrocknet. Das Röntgen-Pulver-Diffraktogramm zeigte keinerlei Veränderung, Form B blieb stabil.

c) N-(Aminoiminomethyl)-2-aminoethansäure Form B aus Beispiel 2.1 wurde zu einer 10 %-igen Suspension in Wasser angesetzt. Diese Suspension wurde 2 Stunden bei 80 °C gerührt. Dann wurde abgekühlt, der Feststoff abfiltriert und getrocknet. Die Röntgen-Pulver-Diffraktometrie ergab, dass ein Gemisch aus Kristallform A und B vorlag.

d) N-(Aminoiminomethyl)-2-aminoethansäure Form B aus Beispiel 2.1 wurde bei 80 °C in Wasser gelöst, durch Abkühlen der Lösung großteils wieder auskristallisiert, abfiltriert und getrocknet. Die Röntgen-Pulver-Diffraktometrie ergab reine Kristallform A.

N-(Aminoiminomethyl)-2-aminoethansäure Form B ist also in fester Form sehr beständig, hat jedoch die Tendenz, über die Wasser-haltige Lösung in Kristallform A überzugehen. Auch dieses Verhalten bestätigt die metastabile Kristallstruktur von Form B.

7.8 Physikalische Eigenschaft von Gemischen Form A und Form B

In Beispiel 7.6 wurde die Schüttdichte von GAA Form A zu 0,37 g/cm$^3$ und diejenige von GAA Form B zu 0,62 g/cm$^3$ ermittelt. Ausgehend von reinen Substanzproben von GAA Form A bzw. Form B wurden Gemische der beiden Formen eingewogen und durch Schütteln (nicht Mahlen oder Mörsern!) gemischt. Die Schüttdichten der so hergestellten Kristallgemische wurden bestimmt.

Tabelle 5: Schüttdichte im Kristallgemisch

| Gewichtsanteil Form A | Gewichtsanteil Form B | Schüttdichte |
|---|---|---|
| 100 % | 0% | 0,62 g/cm$^3$ |
| 75 % | 25 % | 0,59 g/cm$^3$ |
| 50 % | 50 % | 0,53 g/cm$^3$ |
| 25 % | 75 % | 0,41 g/cm$^3$ |
| 0% | 100 % | 0,37 g/cm$^3$ |

Es zeigt sich, dass mit steigenden Anteil GAA Form B die Schüttdichte steigt, wobei ab 50 % Form B die Schüttdichte vorteilhaft über den arithmetischen Mittelwert der beiden Endglieder liegt (vgl. auch Figur 8).

**Patentansprüche**

1. Verfahren zur Herstellung von N-(Aminoiminomethyl)-2-aminoethansäure enthaltend: N-(Aminoiminomethyl)-2-aminoethansäure in einer thermodynamisch metastabilen Kristallmodifikation, wobei die thermodynamisch metastabile Kristallmodifikation im Röntgen-Pulver-Diffraktogramm der Kristallmodifikation bei Verwendung von Cu-K$\alpha$-Strahlung die stärksten Reflexbanden bei 2$\Theta$ = 20,2 ° und 23,3 ° und 23,8 ° und 25,3 ° bei einer Messgenauigkeit von +/- 0,2 ° zeigt, **dadurch gekennzeichnet, dass** N-(Aminoiminomethyl)-2-aminoethansäure aus einer Wasser-haltigen Lösung in Gegenwart von mindestens einer Guanidinverbindungen der Formel (I) kristallisiert wird, wobei für Formel (I) gilt:

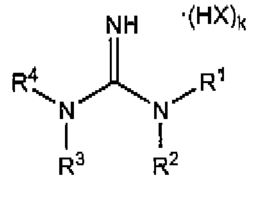

Formel (I)

wobei für die Reste R$^1$, R$^2$, R$^3$, R$^4$ und X, Y sowie den Index k in Formel (I) unabhängig voneinander gilt:

$R^1$, $R^2$ = unabhängig voneinander Wasserstoff, C1- bis C4-Alkyl oder unter Bildung eines Ringes gemeinsam ein Rest der Formel (II), wobei für Formel (II) gilt:

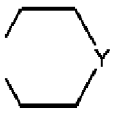

Formel (II)

wobei Y = $CH_2$, 0, NH, $NCH_3$ oder eine Bindung bedeutet,
$R^3$, $R^4$ = unabhängig voneinander Wasserstoff oder C1- bis C4-Alkyl,
X = $CH_3COO$, Cl, Br, $NO_3$, $SO_3(NH_2)$ oder ½ $SO_4$,
k= 0 oder 1,

wobei die Guanidinverbindung der Formel (I) in einer Menge von mindestens 8 Gew.-% (bezogen auf das Gesamtgewicht der Lösung) eingesetzt wird.

**2.** Verfahren nach Anspruch 1, **dadurch gekennzeichnet, dass** die Wasser-haltige Lösung mindestens 40 Gew.-% Wasser (bezogen auf das Gesamtgewicht der Lösung) enthält.

**3.** Verfahren nach Anspruch 1 oder 2, **dadurch gekennzeichnet, dass** N-(Aminoimino-methyl)-2-aminoethansäure in einem ersten Verfahrensschritt in Wasser oder einer Wasser-haltigen Lösung gelöst wird, und die N-(Aminoiminomethyl)-2-aminoethansäure enthaltend N-(Aminoiminomethyl)-2-aminoethansäure in einer thermodynamisch metastabilen Kristallmodifikation in einem zweiten Verfahrensschritt aus der im ersten Verfahrensschritt hergestellten Lösung in Gegenwart der Guanidinverbindung der Formel (I) kristallisiert wird.

**4.** Verfahren nach Anspruch 1 oder 2, **dadurch gekennzeichnet, dass** N-(Aminoimino-methyl)-2-aminoethansäure in einem ersten Verfahrensschritt aus Cyanamid und Glycin in Wasser oder in einer Wasser-haltigen Lösung hergestellt wird, und die N-(Aminoiminomethyl)-2-aminoethansäure enthaltend N-(Aminoiminomethyl)-2-aminoethansäure in einer thermodynamisch metastabilen Kristallmodifikation in einem zweiten Verfahrensschritt aus dem im ersten Verfahrensschritt hergestellten Reaktionsgemisch in Gegenwart der Guanidinverbindung der Formel (I) kristallisiert wird.

**5.** Verfahren nach einem der zuvor genannten Ansprüche, **dadurch gekennzeichnet, dass** die thermodynamisch metastabile Kristallmodifikation die orthorhombische Raumgruppe $P2_12_12_1$ mit Z = 8 mit den Gitterkonstanten a = 7,7685 Å, b = 7,7683 Å und c = 17,4261 Å bei 105 Kelvin und einer Messgenauigkeit von +/- 0,001 Å aufweist.

**6.** Verfahren nach einem der zuvor genannten Ansprüche, **dadurch gekennzeichnet, dass** die Guanidinverbindungen der Formel (I) in einer Menge eingesetzt wird, die 80 % derjenigen Menge entspricht, die bei 25 °C unter Normaldruck in Wasser maximal gelöst werden kann.

**7.** Verfahren nach einem der zuvor genannten Ansprüche, **dadurch gekennzeichnet, dass** N-(Aminoiminomethyl)-2-aminoethansäure im ersten Verfahrensschritt bei einer Temperatur im Bereich von 20 bis 100 °C bei Normaldruck in der Wasser-haltigen Lösung gelöst oder hergestellt wird.

**8.** Kristallgemisch enthaltend N-(Aminoiminomethyl)-2-aminoethansäure in einer thermodynamisch metastabilen Kristallmodifikation, wobei die thermodynamisch metastabile Kristallmodifikation im Röntgen-Pulver-Diffraktogramm der Kristallmodifikation bei Verwendung von Cu-$K\alpha$-Strahlung die stärksten Reflexbanden bei $2\Theta$ = 20,2 ° und 23,3 ° und 23,8 ° und 25,3 ° bei einer Messgenauigkeit von +/- 0,2 ° zeigt, und N-(Aminoiminomethyl)-2-aminoethansäure in einer thermodynamisch stabilen Kristallmodifikation.

**9.** Kristallgemisch nach Anspruch 8, **dadurch gekennzeichnet, dass** das Kristallgemisch N-(Aminoiminomethyl)-2-aminoethansäure in einer thermodynamisch metastabilen Kristallmodifikation und N-(Aminoiminomethyl)-2-aminoethansäure in einer thermodynamisch stabilen Kristallmodifikation in einem Gewichtsverhältnis im Bereich von 0,1 : 9,9 bis 9,9 : 0,1 enthält.

**10.** Futtermittelzusatz für Zucht- und Masttiere umfassend ein Kristallgemisch gemäß Anspruch 8, enthaltend N-(Aminoiminomethyl)-2-aminoethansäure in einer thermodynamisch metastabilen Kristallmodifikation, wobei die thermodynamisch metastabile Kristallmodifikation im Röntgen-Pulver-Diffraktogramm der Kristallmodifikation bei Verwendung von Cu-Kα-Strahlung die stärksten Reflexbanden bei 2Θ = 20,2 ° und 23,3 ° und 23,8 ° und 25,3 ° bei einer Messgenauigkeit von +/- 0,2 ° zeigt, und N-(Aminoiminomethyl)-2-aminoethansäure in einer thermodynamisch stabilen Kristallmodifikation.

**11.** Futtermittelzusatz nach Anspruch 10, **dadurch gekennzeichnet, dass** das Kristallgemisch die N-(Aminoiminomethyl)-2-aminoethansäure in einer thermodynamisch metastabilen Kristallmodifikation und die N-(Aminoiminomethyl)-2-aminoethansäure in einer thermodynamisch stabilen Kristallmodifikation in einem Gewichtsverhältnis im Bereich von 0,1 : 9,9 bis 9,9 : 0,1 enthält.

## Claims

1. A process for preparing N-(aminoiminomethyl)-2-aminoacetic acid containing N-(aminoiminomethyl)-2-aminoacetic acid in a thermodynamically metastable crystal modification, whereby the thermodynamically metastable crystal modification shows in the X-ray powder diffractogram of the crystal modification when using Cu-Kα radiation the strongest reflection bands at 2Θ = 20.2 ° and 23.3 ° and 23.8 ° and 25.3 ° with a measurement accuracy of +/- 0.2 °, **characterized in that** N-(aminoiminomethyl)-2-aminoacetic acid is crystallized from a water-containing solution in the presence of at least one guanidine compound of formula (I), wherein formula (I) represents:

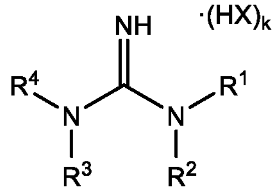

Formula (I)

where radicals $R^1$, $R^2$, $R^3$, $R^4$ and X, Y and index k in formula (I) independently of one another mean:

$R^1$, $R^2$ = independently of one another hydrogen, C1 to C4 alkyl or, with the formation of a ring, together a radical of the formula (II), where the formula (II) is

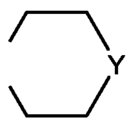

Formula (II)

wherein Y = $CH_2$, O, NH, $NCH_3$ or a bond,
$R^3$, $R^4$= independently hydrogen or C1 to C4 alkyl,
X = $CH_3COO$, Cl, Br, $NO_3$, $SO_3(NH_2)$ or ½ $SO_4$,
k = 0 or 1,

whereby the guanidine compound of formula (I) is used in an amount of at least 8 percent by weight (based on the total weight of the solution).

2. The process according to claim 1, **characterized in that** the water-containing solution contains at least 40% by weight of water (based on the total weight of the solution).

3. The process according to claim 1 or 2, **characterized in that** N-(aminoiminomethyl)-2-aminoacetic acid is dissolved in water or a water-containing solution in a first process step, and the N-(aminoiminomethyl)-2-aminoacetic acid containing N-(aminoiminomethyl)-2-aminoacetic acid in a thermodynamically metastable crystal modification is crystallized in a second process step from the solution prepared in the first process step in the presence of the guanidine compound of formula (I).

4. The process according to claim 1 or 2, **characterized in that** N-(aminoiminomethyl)-2-aminoacetic acid is prepared in a first process step from cyanamide and glycine in water or in a water-containing solution, and the N-(aminoiminomethyl)-2-aminoacetic acid containing N-(aminoiminomethyl)-2-aminoacetic acid in a thermodynamically metastable crystal modification is crystallized in a second process step from the reaction mixture prepared in the first process step in the presence of the guanidine compound of formula (I).

**5.** The process according to any one of the preceding claims, **characterized in that** the thermodynamically metastable crystal modification has the orthorhombic space group $P2_12_12_1$ with Z = 8 with lattice constants a = 7.7685 Å, b = 7.7683 Å and c = 17.4261 Å at 105 Kelvin and a measurement accuracy of +/- 0.001 Å.

**6.** The process according to any one of the preceding claims, **characterized in that** the guanidine compounds of formula (I) are used in an amount corresponding to 80% of the maximum amount which can be dissolved in water at 25°C under normal pressure.

**7.** The process according to any one of the preceding claims, **characterized in that** N-(aminoiminomethyl)-2-aminoacetic acid is dissolved or prepared in the water-containing solution in the first process step at a temperature in the range of 20 to 100 °C at normal pressure.

**8.** A crystal mixture containing N-(aminoiminomethyl)-2-aminoacetic acid in a thermodynamically metastable crystal modification, whereby the thermodynamically metastable crystal modification shows in the X-ray powder diffractogram of the crystal modification when using Cu-Kα radiation the strongest reflection bands at 2 Θ= 20.2 ° and 23.3 ° and 23.8 ° and 25.3 ° with a measurement accuracy of +/- 0.2 °, and N-(aminoiminome-thyl)-2-aminoacetic acid in a thermodynamically stable crystal modification.

**9.** The crystal mixture according to claim 8, **characterized in that** the crystal mixture contains N-(aminoiminomethyl)-2-aminoacetic acid in a thermodynamically metastable crystal modification and N-(aminoiminomethyl)-2-aminoacetic acid in a thermodynamically stable crystal modification in a weight ratio in the range from 0.1 : 9.9 to 9.9 : 0.1.

**10.** A feed additive for breeding and fattening animals comprising a crystal mixture according to claim 8 containing N-(aminoiminomethyl)-2-aminoacetic acid in a thermodynamically metastable crystal modification, whereby the thermodynamically metastable crystal modification shows in the X-ray powder diffractogram of the crystal modification when using Cu-Kα radiation the strongest reflection bands at 2 Θ= 20.2 ° and 23.3 ° and 23.8 ° and 25.3 ° with a measurement accuracy of +/- 0.2 °, and N-(aminoiminomethyl)-2-aminoacetic acid in a thermodynamically stable crystal modification.

**11.** The feed additive according to claim 10, **characterized in that** the crystal mixture contains the N-(aminoiminomethyl)-2-aminoacetic acid in a thermodynamically metastable crystal modification and the N-(aminoiminomethyl)-2-aminoacetic acid in a thermodynamically stable crystal modification in a weight ratio in the range from 0.1 : 9.9 to 9.9 : 0.1.

## Revendications

**1.** Procédé de préparation d'acide N-(aminoiminométhyl)-2-aminoéthanoïque contenant de l'acide N-(aminoiminométhyl)-2-aminoéthanoïque dans une modification cristalline thermodynamiquement métastable, dans lequel la modification cristalline thermodynamiquement métastable présente dans le diffractogramme de poudre aux rayons X de la modification cristalline, en utilisant un rayonnement Cu-Kα, les bandes de réflexion les plus fortes à 2Θ = 20,2 ° et 23,3 ° et 23,8 ° et 25,3 ° avec une précision de mesure de +/- 0,2°, **caractérisé en ce que** l'acide N-(aminoiminométhyl)-2-aminoéthanoïque est cristallisé à partir d'une solution contenant de l'eau en présence d'au moins un composé de guanidine de formule (I), où pour la formule (I) on a :

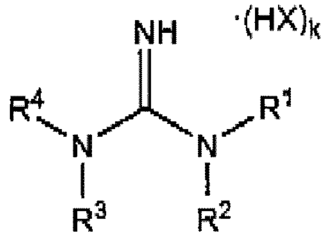

Formulel (I)

où pour les restes $R^1$, $R^2$, $R^3$, $R^4$ et X, Y ainsi que pour l'indice k dans la formule (I), on a indépendamment les uns

des autres :

$R^1$, $R^2$= indépendamment l'un de l'autre hydrogène, C1 - à C4-Alkyl ou, avec formation d'un cycle, ensemble un radical de formule (II), où pour la formule (II) on a :

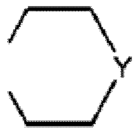

Formule (II)

où Y = $CH_2$, O, NH, $NCH_3$ ou une liaison,
$R^3$, $R^4$ = indépendamment l'un de l'autre, hydrogène ou alkyle en C1 à C4,
X = $CH_3COO$, Cl, Br, $NO_3$, $SO_3(NH_2)$ ou ½ $SO_4$,
k = 0 ou 1,
dans lequel le composé guanidine de formule (I) est utilisé en une quantité d'au moins 8 % en poids (par rapport au poids total de la solution).

2. Procédé selon la revendication 1, **caractérisé en ce que** la solution contenant de l'eau contient au moins 40 % en poids d'eau (par rapport au poids total de la solution).

3. Procédé selon la revendication 1 ou 2, **caractérisé en ce que** l'acide N-(aminoiminométhyl)-2-aminoéthanoïque est dissous dans une première étape du procédé dans de l'eau ou dans une solution contenant de l'eau, et l'acide N-(aminoiminonéthyl)-2-aminoéthanoïque contenant l'acide N-(aminoiminométhyl)-2-aminoéthanoïque dans une modification cristalline thermodynamiquement métastable est cristallisé dans une deuxième étape du procédé à partir de la solution préparée dans la première étape du procédé en présence du composé guanidine de formule (I).

4. Procédé selon la revendication 1 ou 2, **caractérisé en ce que** l'acide N-(aminoiminométhyl)-2-aminoéthanoïque est préparé dans une première étape de procédé à partir de cyanamide et de glycine dans l'eau ou dans une solution contenant de l'eau, et l'acide N-(aminoiminométhyl)-2-aminoéthanoïque contenant l'acide N-(aminoiminométhyl)-2-aminoéthanoïque dans une modification cristalline thermodynamiquement métastable est cristallisé dans une deuxième étape de procédé à partir du mélange réactionnel préparé dans la première étape de procédé en présence du composé de guanidine de formule (I).

5. Procédé selon l'une des revendications précédentes, **caractérisé en ce que** la modification cristalline thermodynamiquement métastable présente le groupe spatial orthorhombique $P2_12_12_1$ avec Z = 8 avec les constantes de réseau a = 7,7685 Å, b = 7,7683 Å et c = 17,4261 Å à 105 Kelvin et une précision de mesure de +/-0,001 Å.

6. Procédé selon l'une des revendications précédentes, **caractérisé en ce que** les composés de guanidine de formule (I) sont utilisés en une quantité qui correspond à 80 % de la quantité qui peut être dissoute au maximum dans l'eau à 25°C sous pression normale.

7. Procédé selon l'une des revendications précédentes, **caractérisé en ce que** l'acide N-(aminoiminométhyl)-2-aminoéthanoïque est dissous ou préparé dans la solution contenant de l'eau dans la première étape du procédé à une température dans la plage de 20 à 100 °C à pression normale.

8. Mélange cristallin contenant de l'acide N-(aminoiminométhyl)-2-aminoéthanoïque dans une modification cristalline thermodynamiquement métastable, dans lequel la modification cristalline thermodynamiquement métastable présentant dans le diffractogramme de poudre aux rayons X de la modification cristalline, en utilisant un rayonnement Cu-K$\alpha$, les bandes de réflexion les plus fortes à $2\Theta$ = 20,2 ° et 23,3 ° et 23,8 ° et 25,3 ° avec une précision de mesure de +/- 0,2 °, et l'acide N-(aminoiminométhyl)-2-aminoéthanoïque dans une modification cristalline thermodynamiquement stable.

9. Mélange cristallin selon la revendication 8, **caractérisé en ce que** le mélange cristallin contient de l'acide N-(aminoiminométhyl)-2-aminoéthanoïque dans une modification cristalline thermodynamiquement métastable et de l'acide N-(aminoiminométhyl)-2-aminoéthanoïque dans une modification cristalline thermodynamiquement stable dans un rapport pondéral dans la plage de 0,1 : 9,9 à 9,9 : 0,1.

**10.** Additif alimentaire pour animaux d'élevage et d'engraissement comprenant un mélange cristallin selon la revendication 8, contenant de l'acide N-(aminoiminométhyl)-2-aminoéthanoïque dans une modification cristalline thermodynamiquement métastable, dans lequel la modification cristalline thermodynamiquement métastable présente dans le diffractogramme de poudre aux rayons X de la modification cristalline, en utilisant un rayonnement Cu-K$\alpha$, les bandes de réflexion les plus fortes à $2\Theta$ = 20,2 ° et 23,3 ° et 23,8 ° et 25,3 ° avec une précision de mesure de +/- 0,2 °, et l'acide N-(aminoiminométhyl)-2-aminoéthanoïque dans une modification cristalline thermodynamiquement stable.

**11.** Additif alimentaire selon la revendication 10, **caractérisé en ce que** le mélange cristallin contient l'acide N-(aminoiminométhyl)-2-aminoéthanoïque dans une modification cristalline thermodynamiquement métastable et l'acide N-{aminoiminométhyl)-2-aminoéthanoïque dans une modification cristalline thermodynamiquement stable dans un rapport pondéral dans la plage de 0,1 : 9,9 à 9,9 : 0,1.

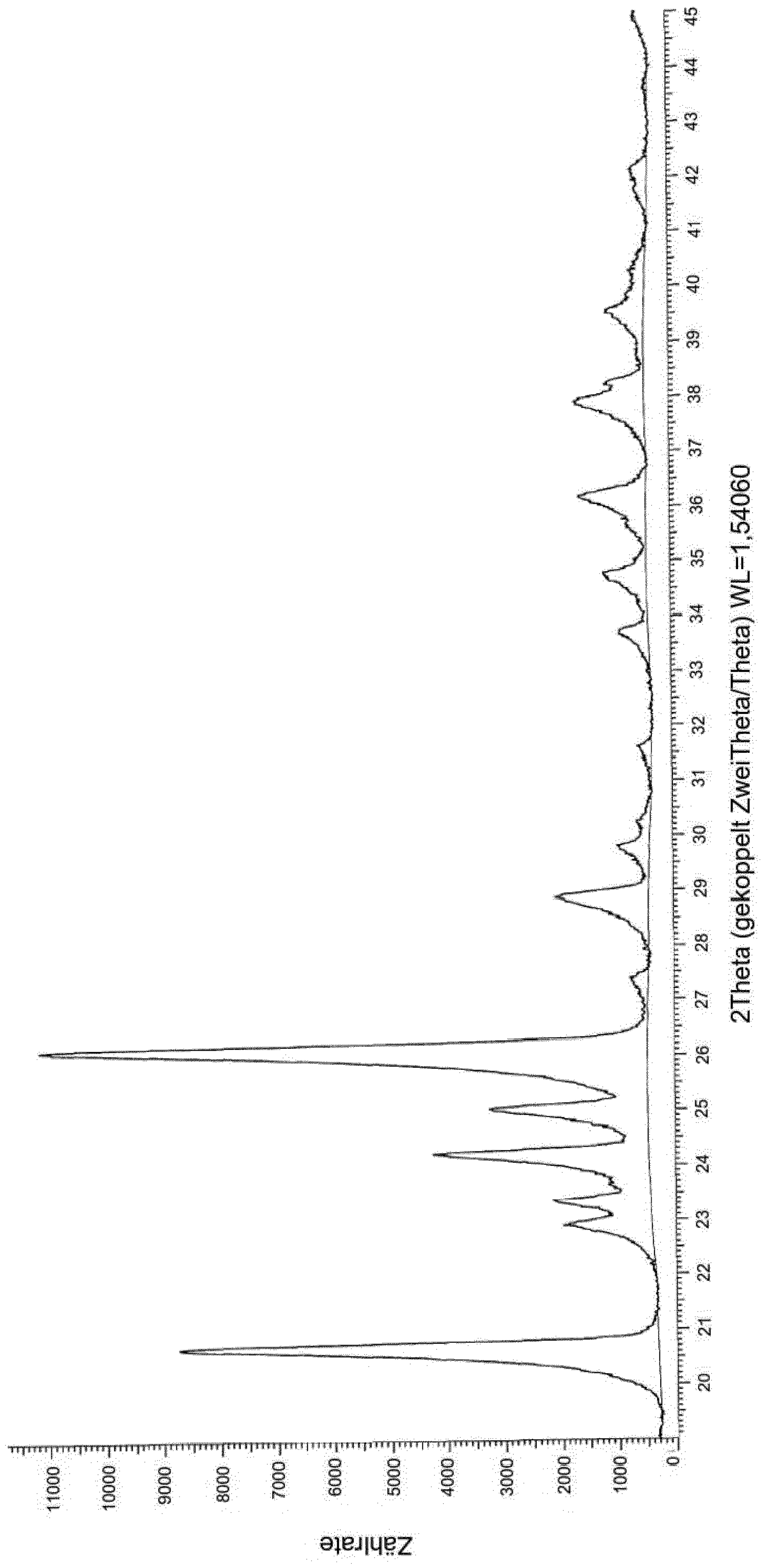
Zählrate
0
1000
2000
3000
4000
5000
6000
7000
8000
9000
10000
11000
20
21
22
23
24
25
26
27
28
29
30
31
32
33
34
35
36
37
38
39
40
41
42
43
44
45
2Theta (gekoppelt ZweiTheta/Theta) WL=1,54060

Figur 1

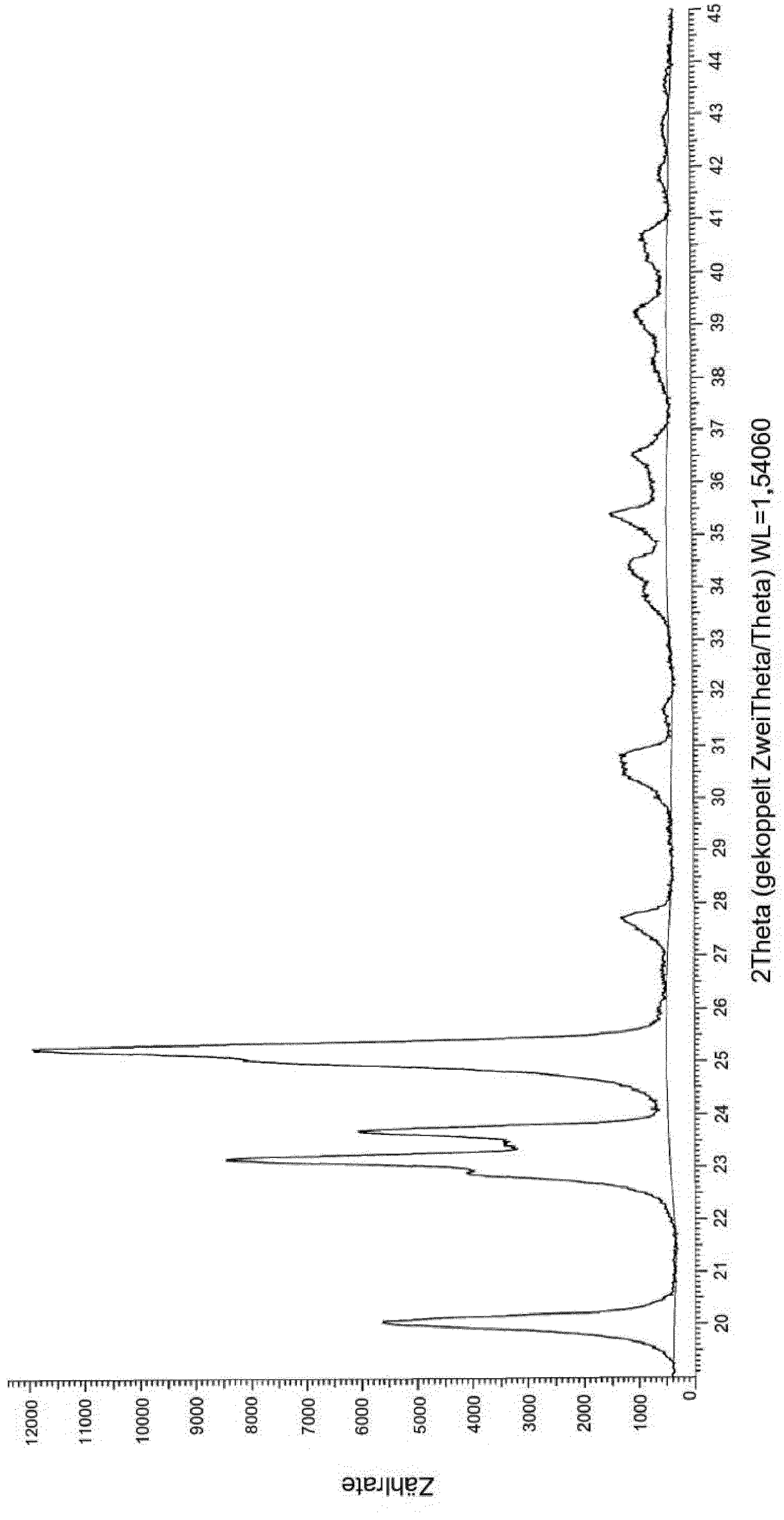
Zählrate
0
1000
2000
3000
4000
5000
6000
7000
8000
9000
10000
11000
12000
20
21
22
23
24
25
26
27
28
29
30
31
32
33
34
35
36
37
38
39
40
41
42
43
44
45
2Theta (gekoppelt ZweiTheta/Theta) WL=1,54060

Figur 2

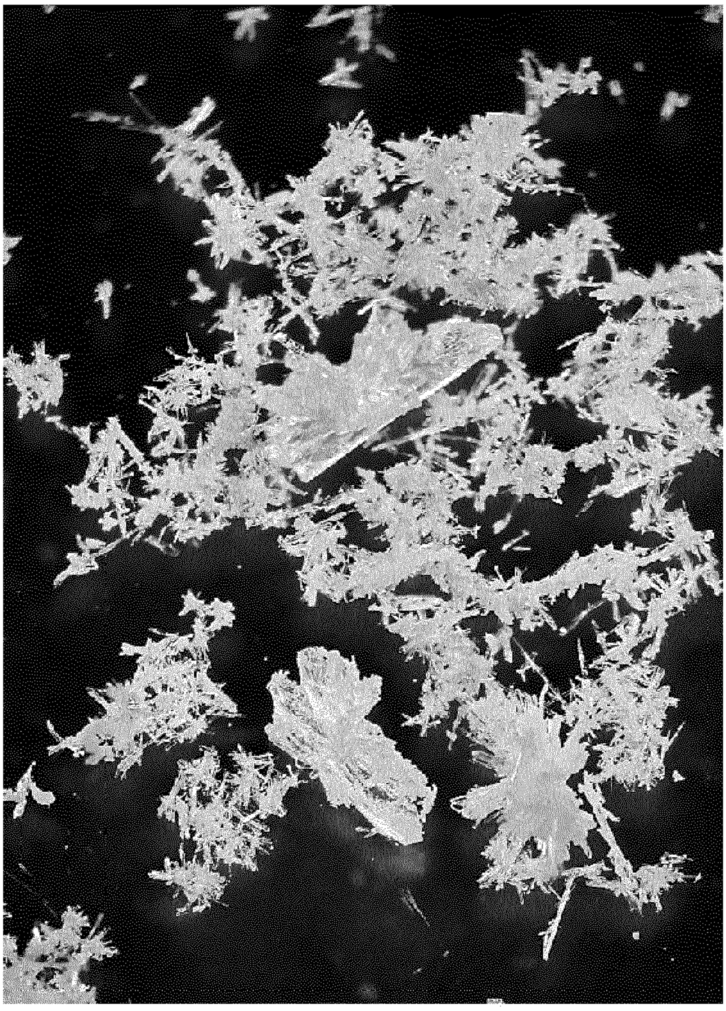

Figur 3

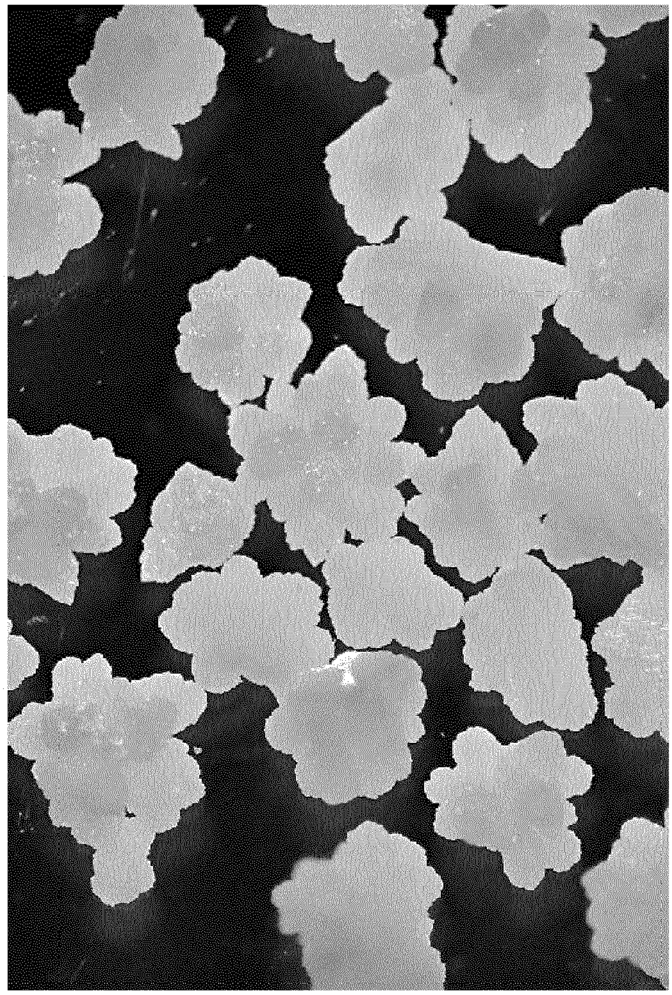

Figur 4

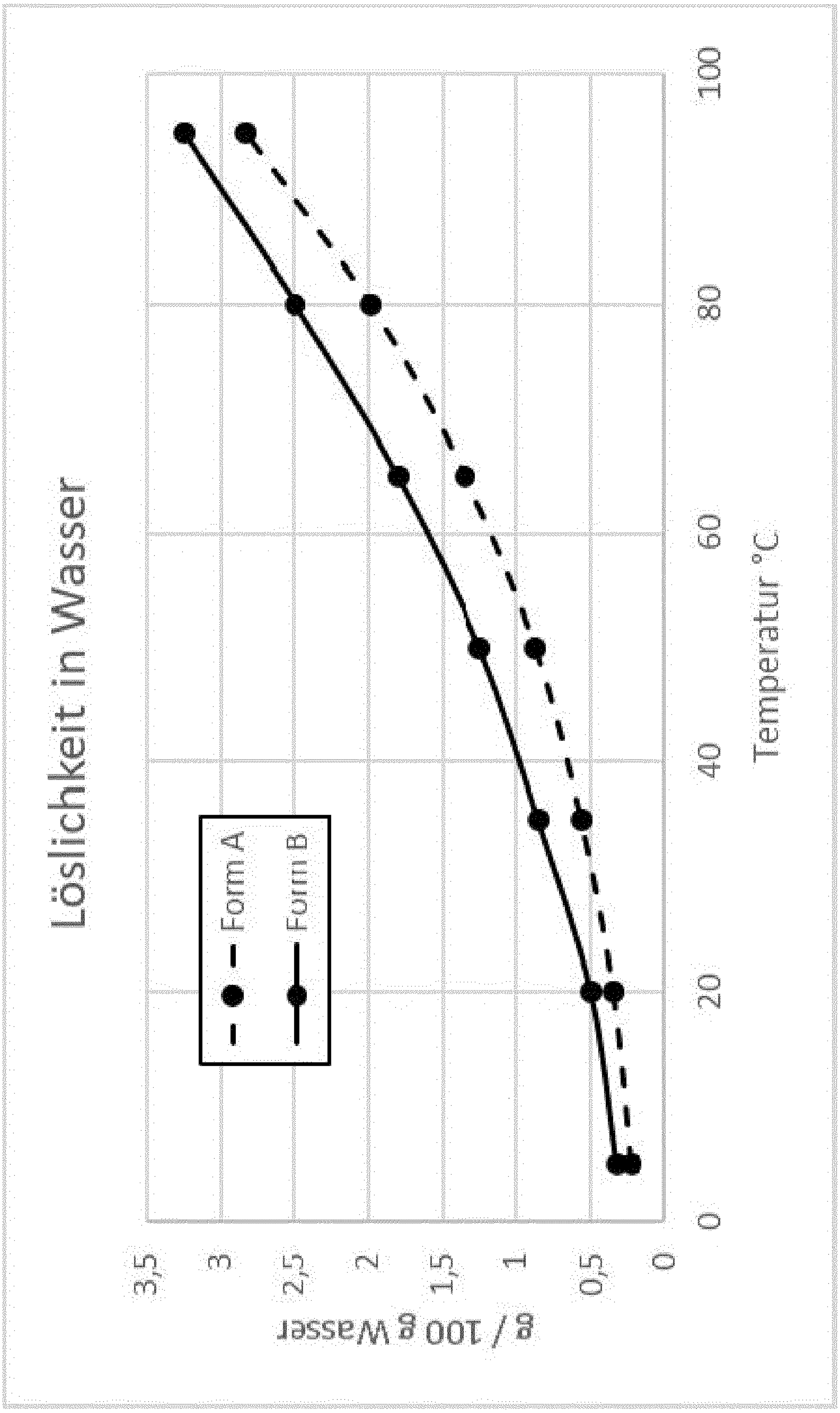
Löslichkeit in Wasser
Form A
Form B
g / 100 g Wasser
3,5
3
2,5
2
1,5
1
0,5
0
0
20
40
60
80
100
Temperatur °C

Figur 5

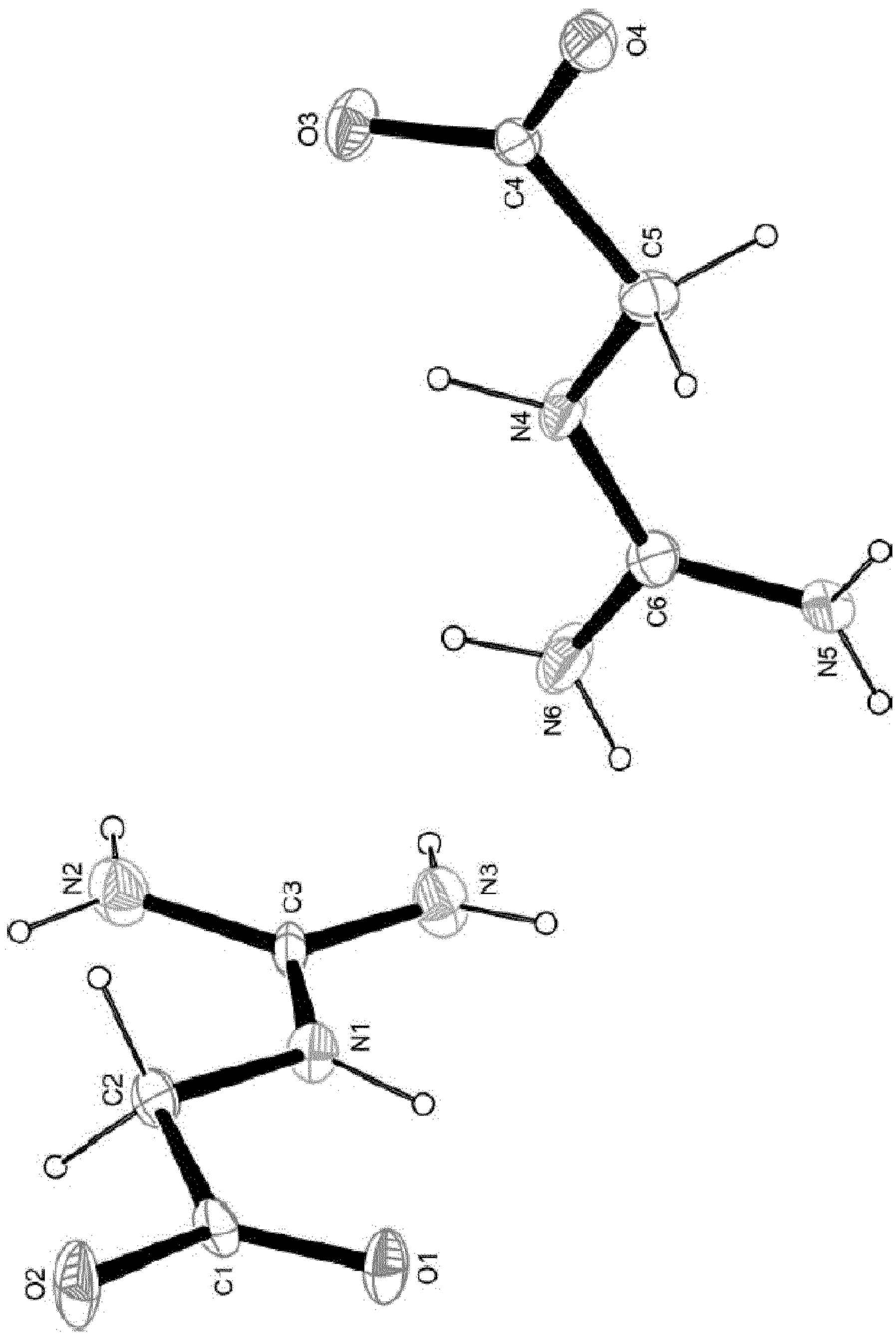

Figur 6

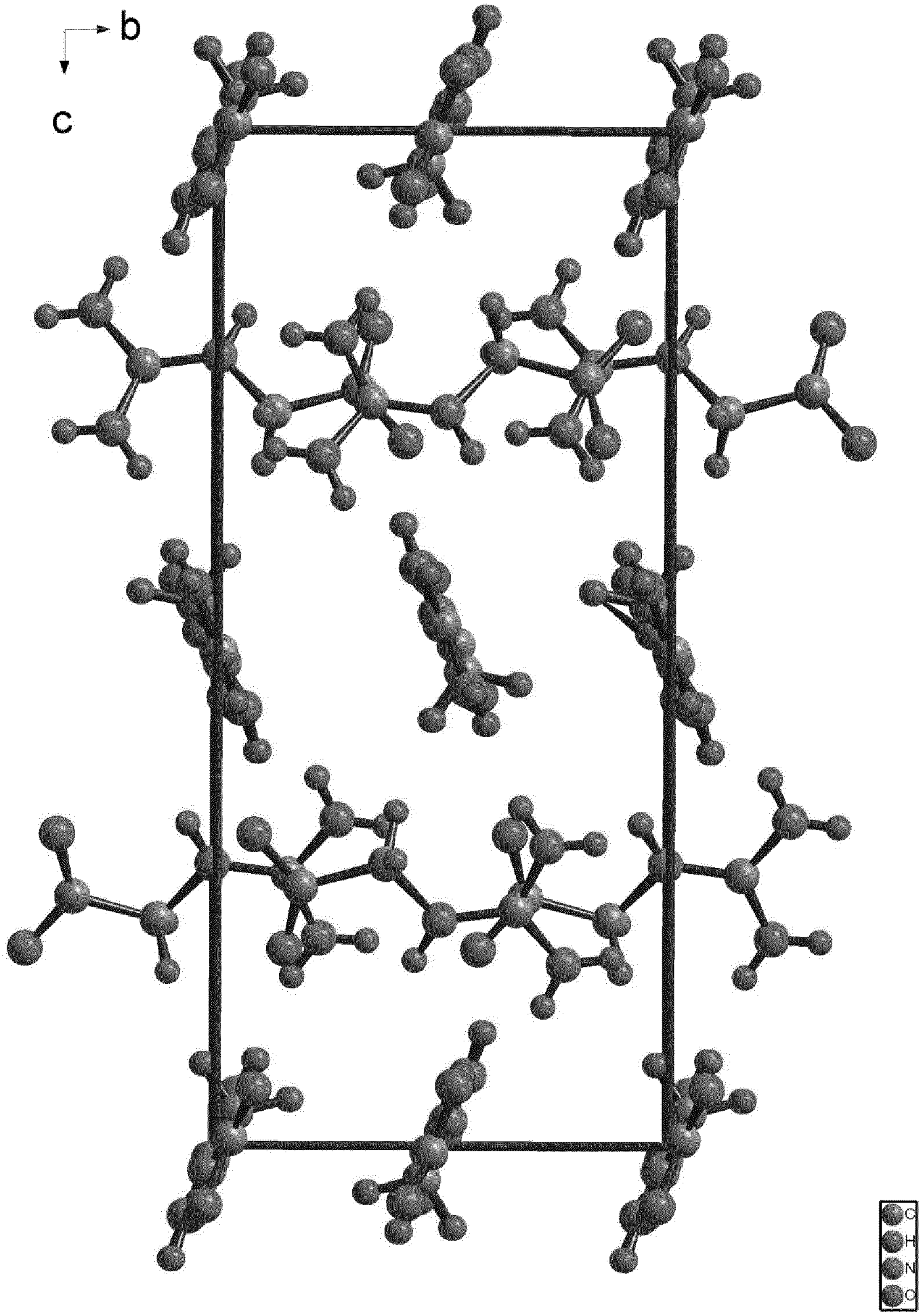


Figur 7

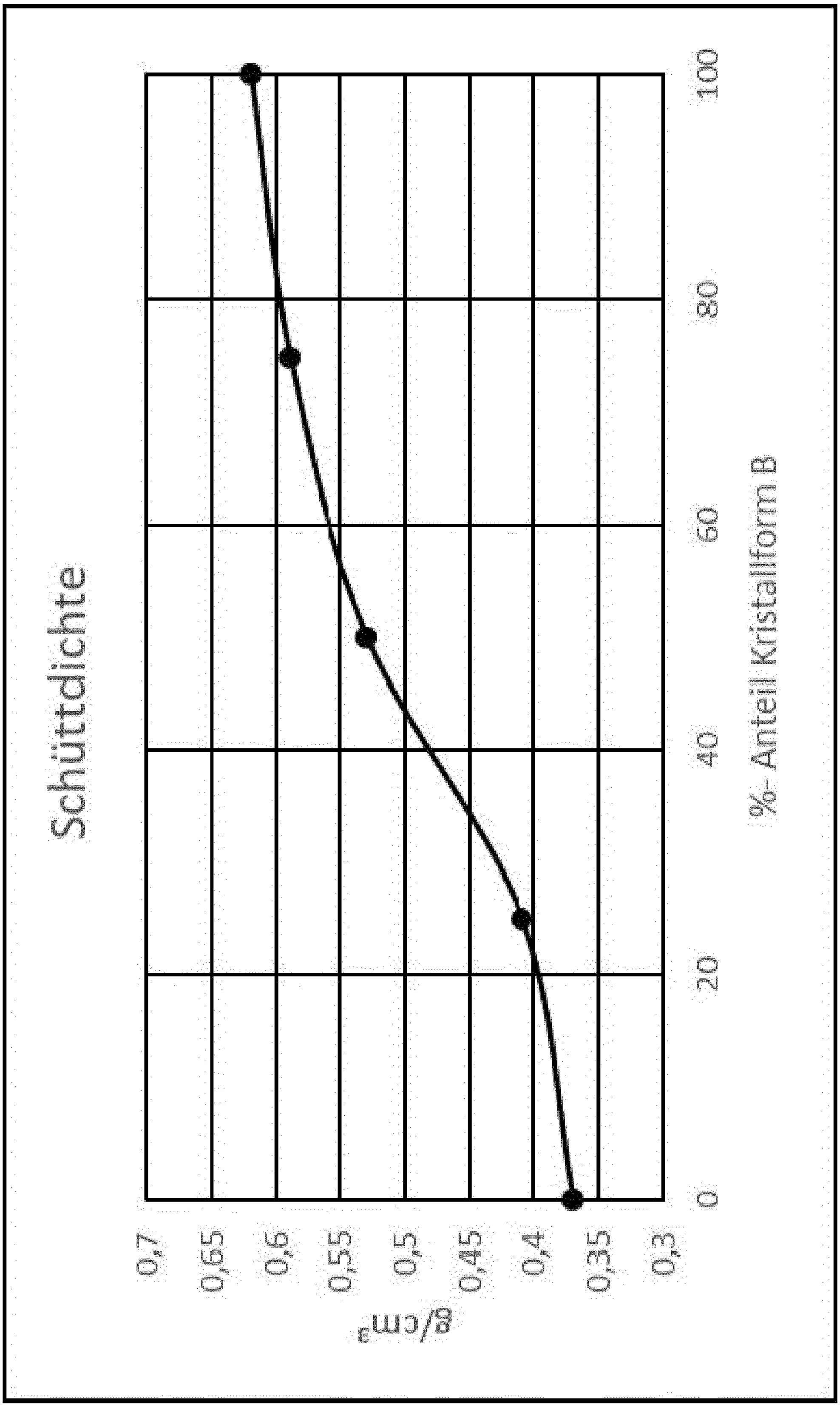
Schüttdichte
g/cm³
0,7
0,65
0,6
0,55
0,5
0,45
0,4
0,35
0,3
0
20
40
60
80
100
%- Anteil Kristallform B

Figur 8

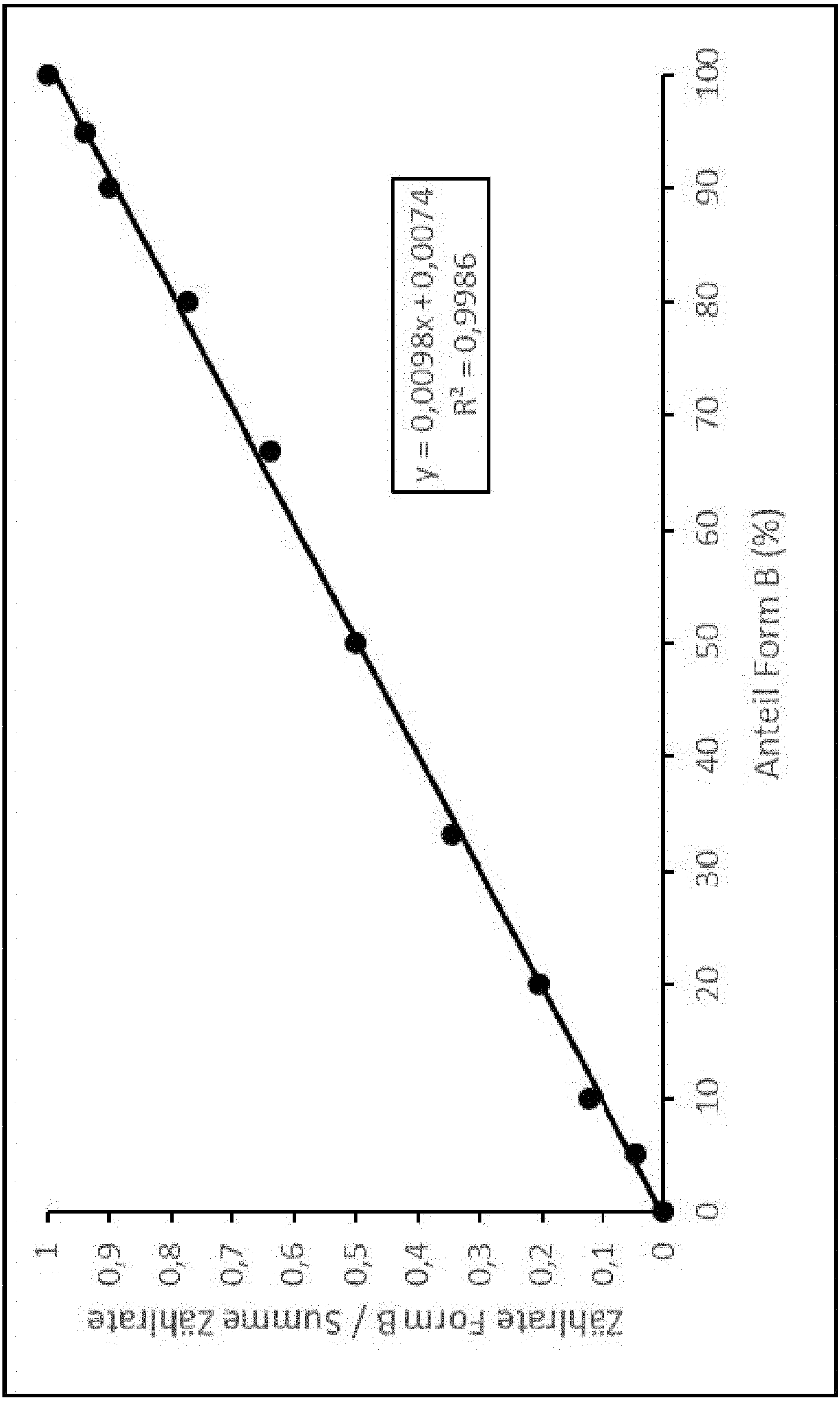
y = 0,0098x + 0,0074
R² = 0,9986
Zählrate Form B / Summe Zählrate
1
0,9
0,8
0,7
0,6
0,5
0,4
0,3
0,2
0,1
0
Anteil Form B (%)
0
10
20
30
40
50
60
70
80
90
100

Figur 9

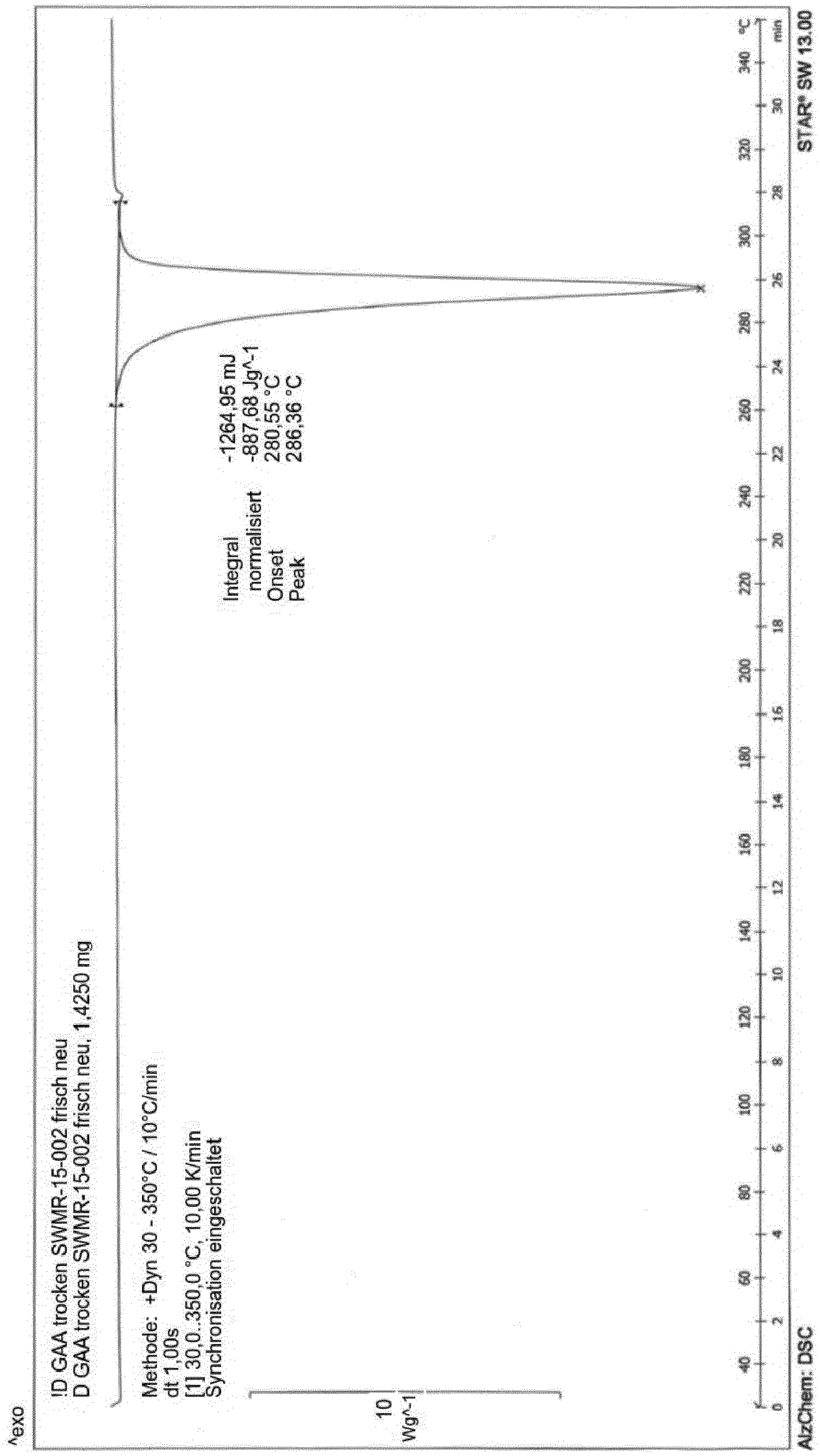
^exo
!D GAA trocken SWMR-15-002 frisch neu
D GAA trocken SWMR-15-002 frisch neu, 1,4250 mg
Methode: +Dyn 30 - 350°C / 10°C/min
dt 1,00s
[1] 30,0..350,0 °C, 10,00 K/min
Synchronisation eingeschaltet
10
Wg^-1
Integral -1264,95 mJ
normalisiert -887,68 Jg^-1
Onset 280,55 °C
Peak 286,36 °C
40 60 80 100 120 140 160 180 200 220 240 260 280 300 320 340 °C
0 2 4 6 8 10 12 14 16 18 20 22 24 26 28 30 min
AlzChem: DSC
STAR® SW 13.00

Figur 10

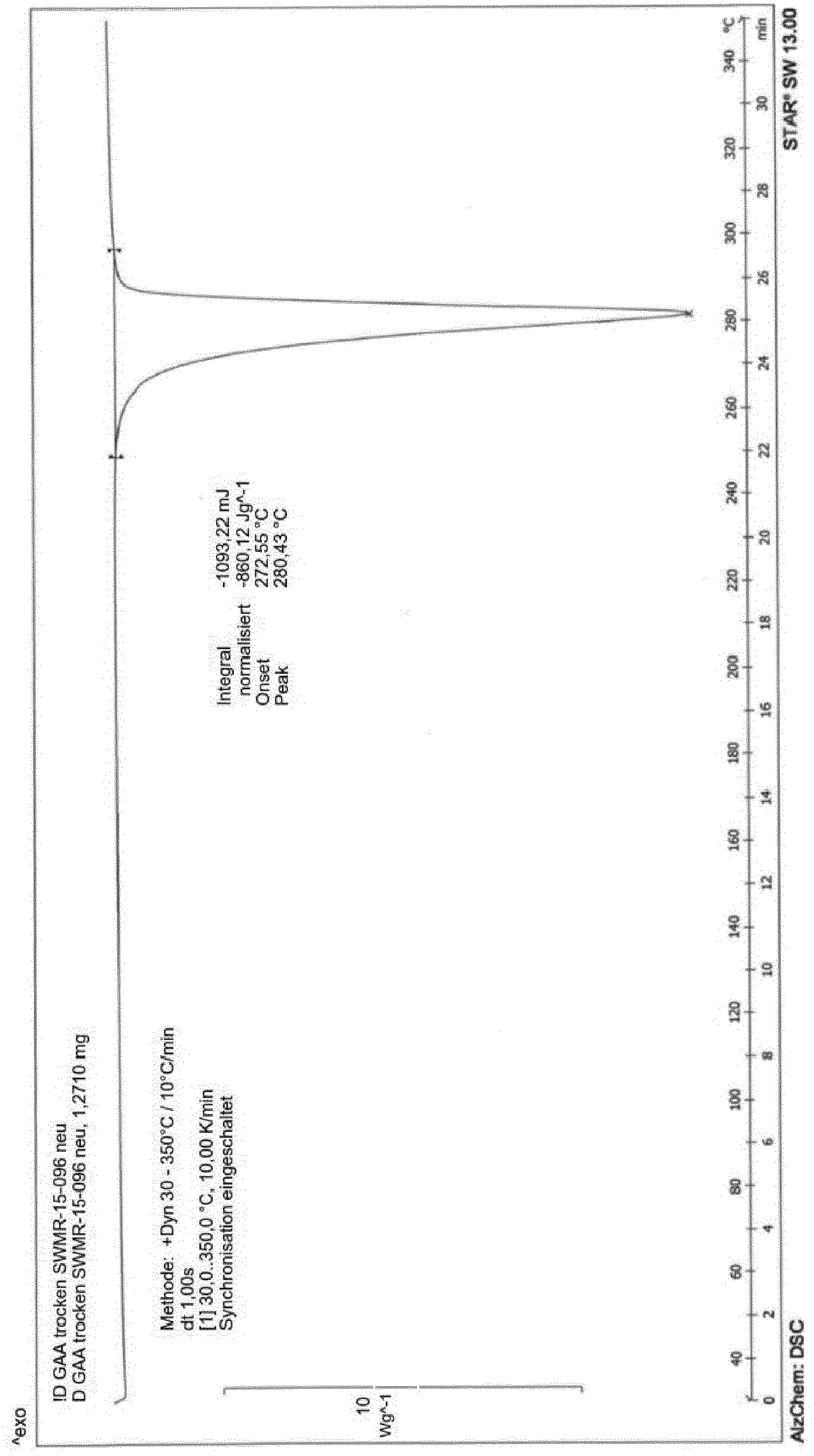
^exo
!D GAA trocken SWMR-15-096 neu
D GAA trocken SWMR-15-096 neu, 1,2710 mg
Methode: +Dyn 30 - 350°C / 10°C/min
dt 1,00s
[1] 30,0..350,0 °C, 10,00 K/min
Synchronisation eingeschaltet
Integral -1093,22 mJ
normalisiert -860,12 Jg^-1
Onset 272,55 °C
Peak 280,43 °C
10
Wg^-1
40 60 80 100 120 140 160 180 200 220 240 260 280 300 320 340 °C
0 2 4 6 8 10 12 14 16 18 20 22 24 26 28 30 min
AlzChem: DSC
STAR® SW 13.00

Figur 11

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2000059528 A
- JP 60054320 B
- CN 106361736
- WO 2005120246 A
- WO 2008092591 A
- DE 102007053369
- US 2654779 A
- US 2620354 A
- CN 101525305
- WO 2009012960 A


### In der Beschreibung aufgeführte Nicht-Patentliteratur


- *CHEMICAL ABSTRACTS,* 352-97-6
- **DU, SHUO.** *Journal of Materials Science,* 2018, vol. 53 (1), 215-229
- **LOPES DE MIRANDA.** *Polyhedron,* 2003, vol. 22 (2), 225-233
- **SINGH, PADMAKSHI.** *Oriental Journal of Chemistry,* 2008, vol. 24 (1), 283-286
- **STRECKER, M.** *Jahresber. Fortschr. Chem. Verw,* 530
- **G.P. JONES ; P. J. PAULING.** Kristall-und Molekularstruktur von Guanidinoessigsäure. *Journal of Crystal and Molecular Structure,* 1979, vol. 9 (5), 273-279
- **S. GUHA.** Kristall- und Molekularstruktur von Glycocyamin. *Acta Cryst,* 1973, vol. B29, 2163-2166
- **VON SANKARANANDA GUHA.** Kristallstruktur wurde von drei Autorengruppen beschrieben. *Acta Cryst.,* 1973, vol. B29, 2163
- **VON PAR J. BERTHOU.** *Acta Cryst,* 1976, vol. B32, 1529
- **WEI WANG.** *Tetrahedron Letters,* 2015, vol. 56, 2684